# FASCICULE DE BREVET EUROPEEN

(11) **EP 1 146 044 B1**
(45) Date de publication et mention de la délivrance du brevet: **01.09.2004**
(21) Numéro de dépôt: 01400939.3
(22) Date de dépôt: 12.04.2001
(51) Int. Cl.: C07D 401/12, A61K 31/4439, A61P 25/00

(54) **Dérivés de cyclobuta-indole carboxamide agissant sur le système nerveux central, leur procédé de préparation et les compositions pharmaceutiques qui les contiennent**
ZNS wirksame Cyclobuta-Indole-Carboxamide, Verfahren zu ihrer Herstellung und diese enthaltende pharmazeutische Präparate
CNS active cyclobuta-indole carboxamide derivatives, processes for their preparation and pharmaceutical compositions containing them

(30) Priorité: 13.04.2000 FR 0004743
(43) Date de publication de la demande: 17.10.2001
(73) Titulaire: LES LABORATOIRES SERVIER, 92200 Neuilly sur Seine (FR)
(72) Inventeur: Peglion, Jean-Louis, 78110 Le Vesinet (FR); Goument, Bertrand, 78220 Viroflay (FR); Millan, Mark, 78230 Le Pecq (FR); Lejeune, Françoise, 92210 Saint Cloud (FR); Cussac, Didier, 78400 Chatou (FR)

(56) Documents cités:
- WO-A-95/29177
- WO-A-96/23783

## Description

La présente invention concerne de nouveaux dérivés de cyclobuta-indole carboxamide, leur procédé de préparation et les compositions pharmaceutiques qui les contiennent.

Les composés de la présente invention sont utiles dans le traitement des maladies du système nerveux central telles que l'anxiété, les attaques de panique, les troubles obsessionnels compulsifs, les phobies, les troubles impulsifs, l'abus de drogue, les troubles de la cognition, les psychoses, les dépressions et les troubles de l'humeur.

De nombreux dérivés polycycliques et hétérocycliques contenant une fonction urée ont été décrits dans la littérature comme étant des antagonistes de divers récepteurs sérotoninergiques, leur conférant ainsi une utilité pour le traitement des maladies du système nerveux central. C'est le cas plus particulièrement des demandes de brevets WO 95/29177, WO 96/23783 et WO 98/47868. Le brevet US 5,514,690 décrit, quant à lui, des dérivés d'aminocarbonylquinoline ou indoline et les revendique pour leur propriété d'activation des pompes à potassium.

Les composés de la présente invention, outre le fait qu'ils soient nouveaux, se sont révélés très actifs pour le traitement des maladies du système nerveux central, et plus particulièrement ont démontrés une forte activité dans le test de conflit de Vogel chez le rat, ainsi que dans le test d'enfouissement de billes chez la souris. Les résultats obtenus dans le premier test permettent de proposer l'utilisation des composés de l'invention dans le traitement des phénomènes cliniques liés à l'anxiété, et ceux obtenus dans le second test démontrent le fort potentiel thérapeutique des composés de l'invention dans le traitement des maladies liées aux troubles de l'humeur.

Plus particulièrement, la présente invention concerne les composés de formule **(I) :** 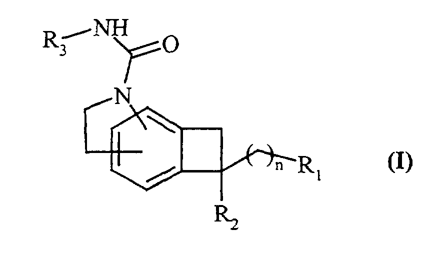 dans laquelle :
**n** représente un entier compris entre 0 et 6,
**R**_{**1**} représente un groupement choisi parmi hydrogène, hydroxy, cyano, alkoxy (C₁-C₆) linéaire ou ramifié, alkoxycarbonyle (C₁-C₆) linéaire ou ramifié, carboxy, aminocarbonyle (la partie amino étant éventuellement substituée par un ou deux groupements, identiques ou différents, choisis parmi alkyle (C₁-C₆) linéaire ou ramifié, aryle et arylalkyle (C₁-C₆) linéaire ou ramifié), et NR₄R₅ dans lequel R₄ et R₅, identiques ou différents, représentent un groupement choisi parmi alkyle (C₁-C₆) linéaire ou ramifié, aryle, arylalkyle (C₁-C₆) linéaire ou ramifié, hétéroaryle, hétéroarylalkyle (C₁-C₆) linéaire ou ramifié, cycloalkyle, cycloalkylalkyle (C₁-C₆) linéaire ou ramifié, alkényle (C₂-C₆) linéaire ou ramifié, et alkynyle (C₂-C₆) linéaire ou ramifié,
**R**_{**2**} représente un groupement choisi parmi atome d'hydrogène, groupement alkyle (C₁-C₆) linéaire ou ramifié, hydroxyméthyle, groupement de formule 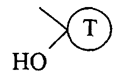 et -U-V-W
   dans lesquels :
   * T représente un groupement cycloalkyle (C₃-C₁₂) monocyclique ou polycyclique, dont l'un des atomes de carbone du cycloalkyle peut éventuellement être remplacé par un groupement choisi parmi atome d'oxygène, atome de sélénium, groupement de formule S(O)ₚ dans laquelle p représente un entier compris entre 0 et 2 inclus, et groupement de formule SiR₆R₇ dans laquelle R₆ et R₇, identiques ou différents, représentent un groupement alkyle (C₁-C₆) linéaire ou ramifié,
   * U représente une liaison ou un groupement méthylène,
   * V représente une liaison, un atome d'oxygène ou un groupement S(O)_{q} dans lequel q est un entier compris entre 0 et 2 inclus,
   * W représente un groupement choisi parmi aryle, arylalkyle (C₁-C₆) linéaire ou ramifié, cycloalkyle, et cycloalkylalkyle (C₁-C₆) linéaire ou ramifié,
**R**_{**3**} représente un groupement choisi parmi atome d'hydrogène, groupement alkyle (C₁-C₆) linéaire ou ramifié, aryle, et hétéroaryle,
leurs isomères ainsi que leurs sels d'addition à un acide ou à une base pharmaceutiquement acceptable.

Par groupement aryle, on comprend un groupement choisi parmi phényle, biphényle, naphtyle, dihydronaphtyle, tétrahydronaphtyle, indanyle, indényle, et benzocyclobutyle, chacun de ces groupements étant éventuellement substitué par un ou plusieurs groupements, identiques ou différents, choisis parmi atomes d'halogène, groupements alkyle (C₁-C₆) linéaire ou ramifié, hydroxy, alkoxy (C₁-C₆) linéaire ou ramifié, nitro, cyano, trihalogénoalkyle (C₁-C₆) linéaire ou ramifié, amino, monoalkylamino, dialkylamino (C₁-C₆) linéaire ou ramifié, trihalogénoalkoxy (C₁-C₆) linéaire ou ramifié, aminoalkyl(C₁-C₆)aminocarbonyle (les atomes d'azote de chacune des parties amino étant éventuellement substitués par des groupements alkyle (C₁-C₆) linéaires ou ramifiés, identiques ou différents), pyridinyle, pyridinyloxy, pyridinyloxyméthyle, ces trois derniers groupements étant éventuellement substitués par un groupement alkyle (C₁-C₆) linéaire ou ramifié.

Par groupement hétéroaryle, on comprend un système monocyclique aromatique ou bicyclique dont l'un des cycles est aromatique, l'autre cycle étant aromatique ou partiellement hydrogéné, de 5 à 12 chaînons, contenant un, deux ou trois hétéroatomes, identiques ou différents, choisis parmi oxygène, azote et soufre, chacun de ces groupements étant éventuellement substitué par un ou plusieurs groupements, identiques ou différents, choisis parmi les substituants décrits pour le groupement aryle précédemment défini.

Par groupement cycloalkyle, on comprend un système mono- ou polycyclique, de 3 à 12 chaînons, contenant éventuellement une ou plusieurs insaturations, celles-ci ne conférant pas de caractère aromatique audit système cyclique.

Par isomères, on comprend les isomères optiques (énantiomères et diastéréoisomères).

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, oxalique, méthane sulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc...

Selon une variante avantageuse de l'invention, les composés préférés de l'invention sont les composés de formule (I) dans laquelle R₂ représente un atome d'hydrogène.

Selon une autre variante avantageuse de l'invention, les composés préférés de l'invention sont les composés de formule (I) dans laquelle R₂ représente un groupement de formule 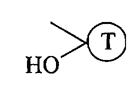 dans laquelle T est tel que défini dans la formule (I), n est égal à 1,
et R₁ représente un groupement cyano ou un groupement amino substitué par un ou deux groupements, identiques ou différents, choisis parmi alkyle (C₁-C₆) linéaire ou ramifié, et arylalkyle (C₁-C₆) linéaire ou ramifié.

D'une autre façon avantageuse, les composés préférés de l'invention sont les composés de formule (I), dans laquelle n est égal à 0, R₁ représente un atome d'hydrogène ou un groupement cyano, et R₂ représente un groupement de formule -U-V-W dans laquelle U représente une liaison simple, V représente un groupement de formule S(O)ₚ avec p tel que défini dans la formule (I) et W représente un groupement aryle.

D'une façon particulièrement avantageuse, les composés préférés de l'invention sont les composés de formule (I) dans laquelle n est égal à 0, R₁ représente un atome d'hydrogène ou un groupement cyano, et R₂ représente un atome d'hydrogène.

Le substituant R₃ préféré selon l'invention est le groupement hétéroaryle, et plus particulièrement le groupement pyridinyle.

Les composés préférés de l'invention sont le :
- *N*-(3-pyridinyl)-2,3,5,6-tétrahydro-1*H*-cyclobuta[*f*]indole-1-carboxamide,
- 5-cyano-N-(3-pyridinyl)-2,3,5,6-tétrahydro-1*H*-cyclobuta[*f*]indole-1-carboxamide,
- 6-cyano-N-(3-pyridinyl)-2,3,5,6-tétrahydro-1*H*-cyclobuta[*f*]indole-1-carboxamide,
- 6-(hydroxyméthyl)-N-(3-pyridinyl)-2,3,5,6-tétrahydro-1*H*-cyclobuta[*f*]indole-1-carboxamide,
- 5-(hydroxyméthyl)-N-(3-pyridinyl)-2,3,5,6-tétrahydro-1*H*-cyclobuta[*f*]indole-1-carboxamide,
- 7-cyano-N-(3-pyridinyl)-1,2,6,7-tétrahydro-3*H*-cyclobuta[*e*]indole-3-carboxamide,
- 7-(hydroxyméthyl)-N-(3-pyridinyl)-2,3,6,7-tétrahydro-1*H*-cyclobuta[*g*]indole-1-carboxamide,
- 6-[(diméthylamino)méthyl]-6-(1-hydroxycyclohexyl)-N-(3-pyridinyl)-2,3,5,6-tétrahydro-1*H*-cyclobuta[*f*]indole-1-carboxamide,
- 6-cyano-6-(phénylsulfanyl)-N-(3-pyridinyl)-2,3,5,6-tétrahydro-1*H*-cyclobuta[f]indole-1-carboxamide,
- 6-cyano-6-cyclohéxyl-N-(3-pyridinyl)-2,3,5,6-tétrahydro-1*H*-cyclobuta[f]indole-1-carboxamide,
- 6-cyclohéxyl-N-(3-pyridinyl)-2,3,5,6-tétrahydro-1*H*-cyclobuta[f]indole-1-carboxamide,
- 6-cyano-N-{6-[(2-méthyl-3-pyridinyl)oxy]-3-pyridinyl}-6-(phénylsulfanyl)-2,3,5,6-tétrahydro-1*H*-cyclobuta[f]indole-1-carboxamide.

Les isomères ainsi que les sels d'addition à un acide ou à une base pharmaceutiquement acceptable des composés préférés font partie intégrante de l'invention.

L'invention concerne aussi le procédé de préparation des composés de formule (I) caractérisé en ce qu'on utilise comme produit de départ un composé de formule **(II)** : 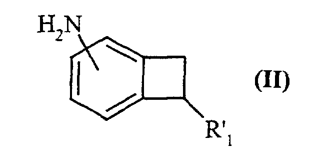 dans laquelle R'₁ représente un groupement choisi parmi atome d'hydrogène, groupement cyano, hydroxyméthylène, carboxy, et alkoxycarbonyle (C₁-C₆) linéaire ou ramifié,
composé de formule (II) qui est mis à réagir dans des conditions d'amination réductrice avec un composé de formule **(III)** :

(AO)₂CHCHO **(III)**

dans laquelle A représente un groupement alkyle (C₁-C₆) linéaire ou ramifié, pour conduire aux composés de formule **(IV)** : 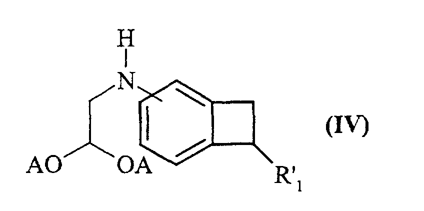 dans laquelle A et R'₁ sont tels que définis précédemment,
composés de formule (IV) qui sont traités par un composé de formule **(V)** :

ClSO₂E **(V)**

dans laquelle E représente un groupement alkyle (C₁-C₄) linéaire ou ramifié, phényle ou p-toluyle, pour conduire aux composés de formule **(VI)** : 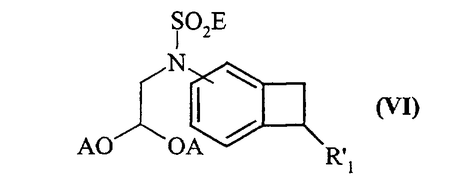 dans laquelle A, E et R'₁ sont tels que définis précédemment,
composés de formule (VI) qui sont cyclisés dans des conditions acides, pour conduire aux composés de formule **(VII)** : 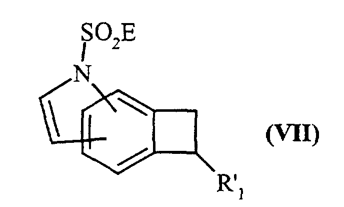 dans laquelle E et R'₁ sont tels que définis précédemment,
composés de formule (VII) qui sont traités soit par un hydroxyde de métal alcalin dans un solvant alcoolique, soit par le sodium dans l'ammoniaque liquide pour conduire aux composés de formule **(VIII)** : 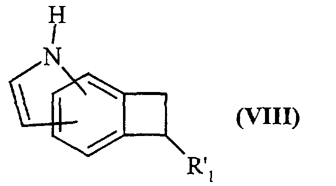 dans laquelle R'₁ est tel que défini précédemment,
composés de formule (VIII) qui sont ensuite réduits selon des conditions classiques de la synthèse organique, pour conduire aux composés de formule **(IX) :** 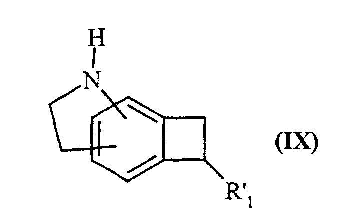 dans laquelle R'₁ est tel que défini précédemment,
composés de formule (IX) qui sont traités par un isocyanate de formule (**X**) :

R₃-N=C=O **(X)**

dans laquelle R₃ est tel que défini dans la formule (I),
pour conduire aux composés de formule **(I/a)**, cas particulier des composés de formule (I) : 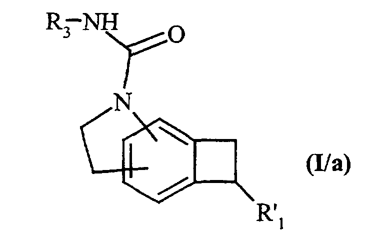 dans laquelle R'₁ et R₃ sont tels que définis précédemment,
ou composés de formule **(IX),** dans le cas particulier où R'₁ représente un groupement cyano, que l'on traite :
* *soit* par une cétone de formule **(XI)** : 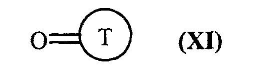 dans laquelle T a les mêmes significations que dans la formule (I), pour conduire aux composés de formule **(XII)** : 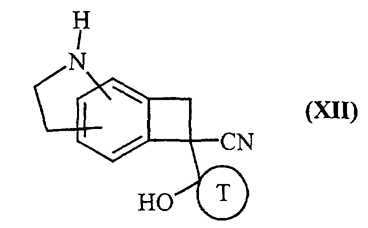 dans laquelle T est tel que défini précédemment,
composés de formule (XII) qui sont ensuite :
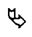 **soit** traités par un isocyanate de formule (X) telle que décrite précédemment, pour conduire aux composés de formule **(I/b),** cas particulier des composés de formule (I), 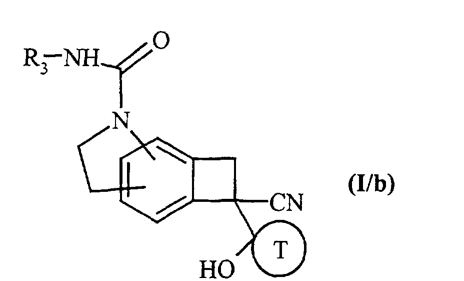 dans laquelle T et R₃ sont tels que définis précédemment,
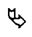 **soit,** après protection de l'amine du groupe indoline, réduits selon les méthodes classiques de la synthèse organique, pour conduire aux composés de formule **(XIII)** : 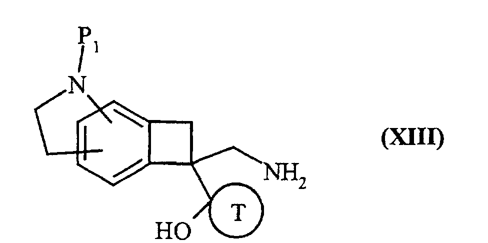 dans laquelle T est tel que défini précédemment et P₁ est un groupement protecteur classique,
composés de formule (XIII) dont la fonction amine primaire est ensuite substituée et transformée en fonction amine secondaire puis tertiaire, en utilisant des méthodes usuelles de la chimie organique, pour conduire aux composés de formule **(XIV)** : 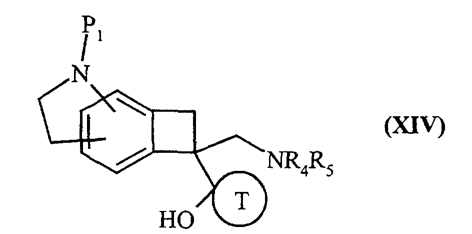 dans laquelle R₄ et R₅ ont les mêmes significations que dans la formule (I) et, T et P₁ sont tels que définis précédemment,
composés de formule (XIV) qui, après déprotection de l'atome d'azote du noyau indoline, sont traités par un composé de formule (X) telle que décrite précédemment, pour conduire aux composés de formule **(I/c)**, cas particulier des composés de formule (I) : 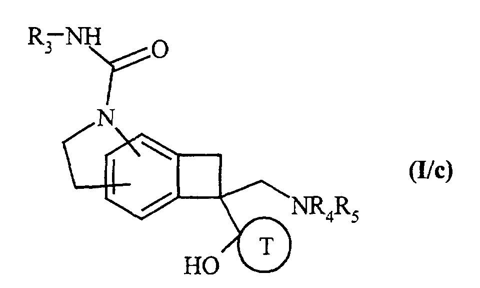 dans laquelle T, R₄, R₅ et R₃ sont tels que définis précédemment,
* *soit* par une base forte ou un alcoolate de métal alcalin, en présence d'un composé de formule **(XV)** :

W₁ - X **(XV)**

dans laquelle W₁ représente un groupement alkyle (C₁-C₆) linéaire ou ramifié, arylalkyle (C₁-C₆) linéaire ou ramifié, cycloalkyle, ou cycloalkylalkyle (C₁-C₆) linéaire ou ramifié, et X représente un groupe partant tel qu'un atome d'halogène, un groupement trifluorométhylsulfonate, mésylate ou tosylate,
pour conduire aux composés de formule **(XVI)** : 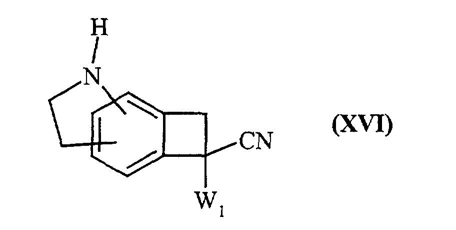 dans laquelle W₁ est tel que défini précédemment,
composés de formule (XVI) qui sont :
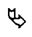 **soit** traités par un composé de formule (X) tel que décrit précédemment, pour conduire aux composés de formule **(I/d),** cas particulier des composés de formule (I) : 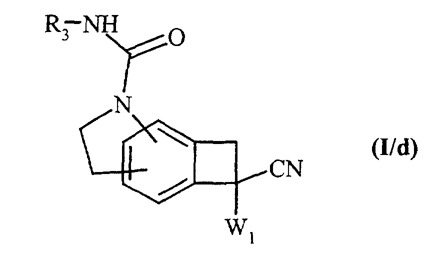 dans laquelle W₁ et R₃ sont tels que définis précédemment,
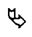 **soit** transformé, après protection de l'atome d'azote du noyau indoline, comme les composés de formule (XII) en amine primaire, secondaire puis tertiaire, pour conduire après déprotection et traitement en présence d'un composé de formule (X) telle que décrite précédemment, aux composés de formule **(I/e),** cas particulier des composés de formule (I) : 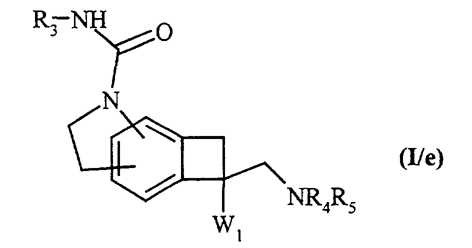 dans laquelle W₁, R₃, R₄ et R₅ sont tels que définis précédemment,
* *soit* par du dibrome dans un solvant organique chloré pour conduire aux composés de formule **(XVII)** : 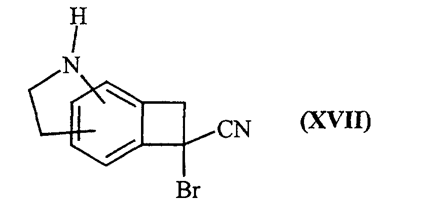 composés de formule (XVII) qui sont mis à réagir avec un composé de formule **(XVIII)** :

W - V₁ - H **(XVIII)**

dans laquelle W a la même signification que dans la formule (I) et V₁ représente un atome d'oxygène ou un atome de soufre,
pour conduire aux composés de formule **(XIX)** : 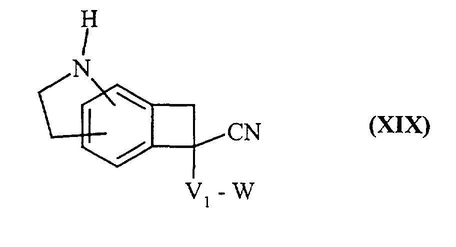 dans laquelle V₁ et W sont tels que définis précédemment,
composés de formule (XIX) qui sont :
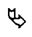 **soit** traités par un composé de formule (X) tel que décrit précédemment, pour conduire aux composés de formule **(I/f)**, cas particulier des composés de formule (I) : 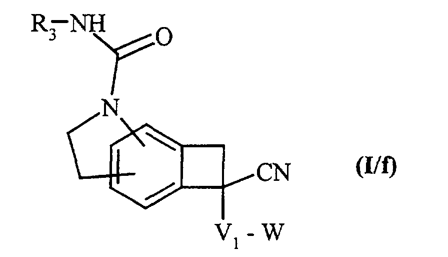 dans laquelle R₃, V₁ et W sont tels que définis précédemment,
composés de formule (I/f), dans le cas particulier où V₁ représente un atome de soufre, qui peuvent être soumis à une oxydation dans des conditions classiques de la synthèse organique, pour conduire aux composés de formule **(I/g),** cas particulier des composés de formule (I) : 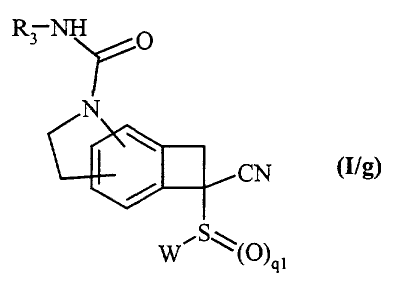 dans laquelle R₃ et W sont tels que définis dans la formule (I) et q₁ est un entier compris entre 1 et 2 inclus,
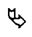 **soit** protégés puis transformés, par la même séquence de réactions que les composés de formule (XII), en amine primaire, secondaire et tertiaire, pour conduire après déprotection et traitement par un composé de formule (X), tel que décrit précédemment, aux composés de formule **(I/h)**, cas particulier des composés de formule (I) : 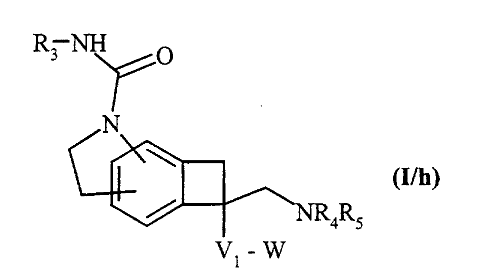 dans laquelle V₁, W, R₃, R₄ et R₅ sont tels que définis précédemment,
composés de formule (I/h) dans le cas particulier où V₁ représente un atome de soufre, qui peuvent être soumis à une oxydation dans des conditions classiques de la synthèse organique, pour conduire aux composés de formule **(I/i)**, cas particulier des composés de formule (I) : 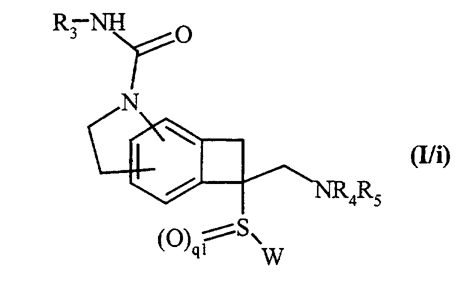 dans laquelle W, R₃, R₄, R₅ et q₁ sont tels que définis précédemment,
* *soit* par un hydrure alcalin dans la diméthylformamide en présence de formaldéhyde, pour conduire aux composés de formule **(XX)** : 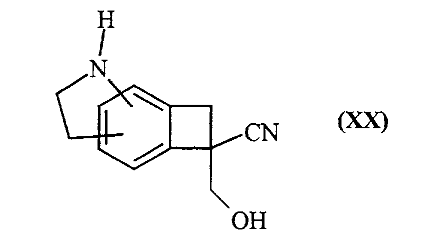 composés de formule (XX) qui sont :
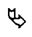 **soit** traités par un composé de formule (X) tel que décrit précédemment, pour conduire aux composés de formule **(I/j),** cas particulier des composés de formule (I) : 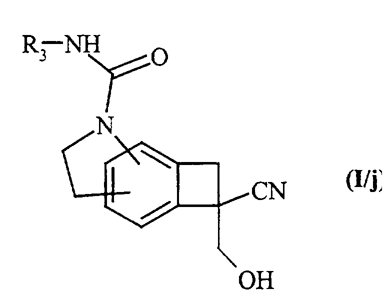 dans laquelle R₃ est tel que défini dans la formule (I),
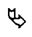 **soit** protégés au niveau de l'atome d'azote du noyau indoline, puis traités selon les conditions de la réaction de Mitsunobu par un composé de formule **(XXI)** :

W - OH **(XXI)**

dans laquelle W est tel que défini dans la formule (I),
pour conduire, après déprotection de l'atome d'azote du noyau indoline, aux composés de formule **(XXII)** : 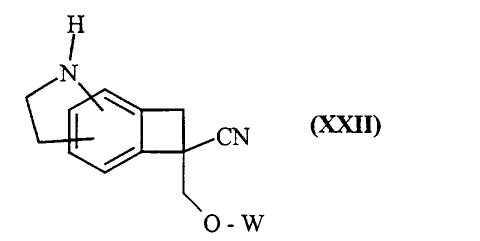 dans laquelle W est tel que défini précédemment,
composés de formule (XXII) qui sont :
◆ ***soit*** traités par un composé de formule (X) tel que décrit précédemment, pour conduire aux composés de formule **(I/k)**, cas particulier des composés de formule (I) : 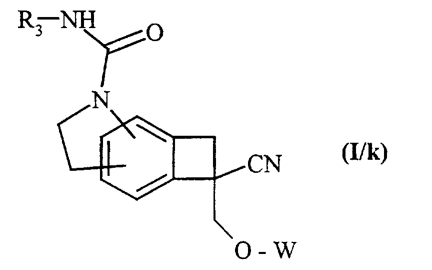 dans laquelle R₃ et W sont tels que définis précédemment,
*◆* ***soit*** protégés puis transformés, par la même séquence de réaction que les composés de formule (XII), en amine primaire, secondaire et tertiaire, pour conduire après déprotection et traitement par un composé de formule (X), tel que décrit précédemment, aux composés de formule **(I/l)**, cas particulier des composés de formule (I) : 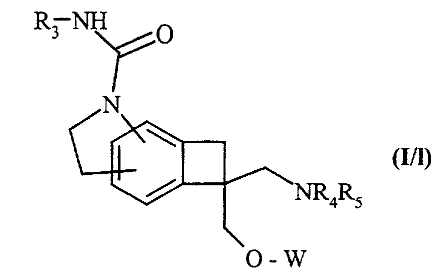 dans laquelle R₃, R₄, R₅ et W sont tels que définis précédemment,
les composés (I/a) à (I/l) forment l'ensemble des composés de l'invention, que l'on purifie le cas échéant, selon une technique classique de purification, qui peuvent, si on le désire, être séparés en leurs différents isomères selon une technique classique de séparation, et que l'on transforme, le cas échéant, en leurs sels d'addition à un acide ou à une base pharmaceutiquement acceptable.

Les composés de formule (II), (III), (V), (X), (XI), (XV), (XVIII) et (XXI) sont soit des produits connus, soit des produits obtenus à partir de substances connues selon des procédés classiques de la chimie organique.

Les composés de la présente invention, de part leurs propriétés pharmacologiques, sont utiles, en tant que médicament, dans le traitement de l'anxiété, des attaques de panique, des troubles obsessionnels compulsifs, des phobies, des troubles impulsifs, de l'abus de drogue, des troubles de la cognition, des psychoses, des dépressions, et des troubles de l'humeur.

La présente invention a également pour objet les compositions pharmaceutiques contenant comme principe actif au moins un composé de formule (I), ses isomères optiques, ses sels d'addition à un acide ou à une base pharmaceutiquement acceptable, seul ou en combinaison avec un ou plusieurs excipients ou véhicules inertes, non toxiques, pharmaceutiquement acceptables.

Parmi les compositions pharmaceutiques selon l'invention, il sera cité plus particulièrement celles qui conviennent pour l'administration orale, parentérale (intraveineuse, intramusculaire ou sous-cutanée), per ou transcutanée, nasale, rectale, perlinguale, oculaire ou respiratoire, et notamment les comprimés simples ou dragéifiés, les comprimés sublinguaux, les gélules, les capsules, les suppositoires, les crèmes, les pommades, les gels dermiques, les préparations injectables ou buvables, les aérosols, les gouttes oculaires ou nasales, etc...

La posologie utile varie selon l'âge et le poids du patient, la voie d'administration, la nature et la sévérité de l'affection, et la prise de traitements éventuels associés et s'échelonne de 0,5 mg à 25 mg en une ou plusieurs prises par jour.

Les exemples suivants illustrent l'invention mais ne la limitent en aucune façon. Les produits de départ utilisés sont des produits connus ou préparés selon des modes opératoires connus. Les différentes préparations conduisent à des intermédiaires de synthèse utiles pour la préparation des composés de l'invention.

Les structures des composés décrits dans les exemples ont été déterminées selon les techniques spectrophotométriques usuelles (infrarouge, résonance magnétique nucléaire, spectrométrie de masse, ...).

Les points de fusion ont été déterminés soit à la platine chauffante de Kofler (K.), soit à la platine chauffante sous microscope (M.K.). Lorsque le composé existe sous forme de sel, le point de fusion donné correspond à celui du produit salifié.

A titre indicatif, la numérotation adoptée pour les systèmes tricycliques est la suivante :

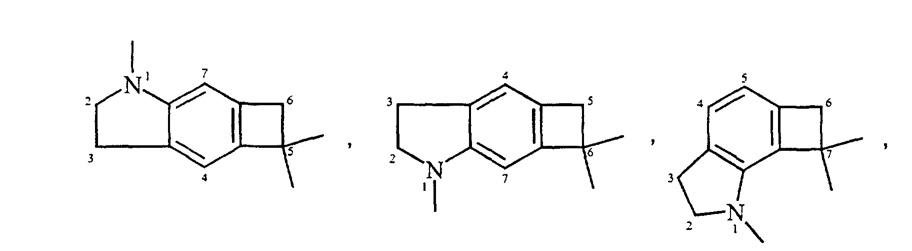

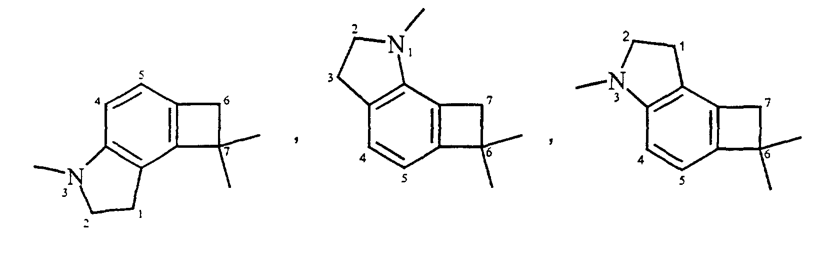

### PREPARATION 1 : 2,3,5,6-Tétrahydro-1H-cyclobuta[f]indole-6-carbonitrile

### Stade 1 : 5-[(2,2-Diméthoxyéthyl)amino]benzocyclobutane-1-carbonitrile

Sur une suspension de 13,5 g de 5-amino-benzocyclobutane-1-carbonitrile dans 400 ml de 1,2-dichloroéthane sont ajoutés par un goutte à goutte rapide, 26,5 ml d'une solution de 2,2-diméthoxy-acétaldéhyde à 45 % dans le tertbutylméthyléther, puis 16 ml d'acide acétique, puis par fraction, 39,7 g de triacétoxyborohydrure de sodium. Après élévation de la température à 29°C, le milieu réactionnel est ramené à température ambiante, agité 1 h 15 puis hydrolysé en versant le milieu sur 500 ml d'une solution aqueuse saturée au NaHCO₃. La phase organique est séparée, lavée à l'eau, et concentrée sous pression réduite pour conduire au produit désiré.

### Stade 2 : 5-[N-(2,2-Diméthoxyéthyl)-N-(méthylsulfonyl)amino]benzocyclobutane-1-carbonitrile

A une solution refroidie à 0°C de 21,6 g du produit obtenu au stade 1, 58 ml de pyridine et 225 ml de dichlorométhane sont ajoutés, en 20 minutes, 10,8 ml de chlorure de mésyle. Après 40 minutes d'agitation à 0°C puis 20 heures à température ambiante, le milieu réactionnel est versé sur 40 ml d'une solution aqueuse saturée en NaHCO₃. Après décantation et extraction par 2 fois 150 ml de dichlorométhane, les phases organiques jointes sont lavées à l'acide chlorhydrique 1N, séchées puis concentrées sous pression réduite pour conduire au produit attendu.

### Stade 3 : 1-(Méthylsulfonyl)-5,6-dihydro-1H-cyclobuta[f]indole-6-carbonitrile

Sur 2,1 l de toluène à reflux sont coulés simultanément en 1 h 15, une solution de 10,9 ml de chlorure de titane dans 450 ml de toluène et une solution de 27,9 g du produit obtenu au stade 2 dans 450 ml de toluène. A la fin de l'addition, on laisse redescendre la température jusqu'à 40°C, puis verse sur 1,8 l d'une solution aqueuse saturée en NaHCO₃. Après décantation, la phase aqueuse est extraite au toluène, les phases organiques sont réunies, lavées, séchées et concentrées. Le résidu est purifié par chromatographie sur gel de silice (dichlorométhane/cyclohexane: 75/25) permettant d'isoler le produit attendu ainsi que son régioisomère.
**Point de fusion : 142-144°C (M.K.)**

### Stade 4 : 5,6-Dihydro-1H-cyclobuta[f]indole-6-carbonitrile

2,6 g du produit obtenu au stade 3 sont introduits dans une solution de 7,7 g de potasse dans 190 ml de méthanol. Après 12 heures à reflux, le méthanol est évaporé et le résidu repris par de l'éther. Après lavage, la phase organique est séchée et concentrée pour conduire au produit attendu.
**Point de fusion : 126-128°C (M.K.)**

### Stade 5 : 2,3,5,6-tétrahydro-1H-cyclobuta[f]indole-6-carbonitrile

3,43 g du produit obtenu au stade 4 sont solubilisés dans 55 ml d'acide acétique. Dans le milieu réactionnel refroidi à 13°C, sont ajoutés, par portions, en 5 minutes, 3,84 g de cyanoborohydrure de sodium. Après retour à température ambiante, l'agitation est maintenue pendant 2 heures, puis le milieu réactionnel est refroidi à 0°C et amené à pH = 11 par addition d'une solution de soude (45 g dans 250 ml d'eau). La solution laiteuse obtenue est extraite à l'éther. Les phases organiques sont lavées, séchées et concentrées pour conduire au produit attendu.
**Point de fusion : 85-87°C (M.K.)**

### PREPARATION 2 : 2,3,6,7-Tétrahydro-1H-cyclobuta[e]indole-7-carbonitrile

### Stade 1 : 6,7-Dihydro-3H-cyclobuta[e]indole-7-carbonitrile

Le produit est obtenu selon le procédé du stade 4 de la préparation 1 en utilisant comme substrat le régioisomère obtenu au stade 3 de la préparation 1.

### Stade 2 : 2,3,6,7-Tétrahydro-1H-cyclobuta[e]indole-7-carbonitrile

Le produit est obtenu, à partir du composé obtenu au stade 1, selon le procédé du stade 5 de la préparation 1.

### PREPARATION 3 : 2,3,5,6-Tétrahydro-1H-cyclobuta[f]indole

### Stade 1 : 4-[(2,2-Diméthoxyéthyl)amino]benzocyclobutane

Une solution de 1 g du produit obtenu au stade 1 de la préparation 1, dans 20 ml de tétrahydrofurane et 0,22 ml d'éthanol anhydre est ajoutée, à -70°C, à 40 ml d'ammoniac liquide. 322 mg de sodium sont alors ajoutés par portions, et l'agitation est maintenue à -70°C pendant 20 minutes. La réaction est stoppée par addition de 1,72 g de NH₄Cl, et on laisse tout l'ammoniac s'évaporer. Le milieu réactionnel est repris par une solution saturée en NH₄Cl puis extrait à l'éther. La phase organique est ensuite séchée puis concentrée pour conduire au produit attendu.

### Stade 2 : 2,3,5,6-Tétrahydro-1H-cyclobuta[f]indole

Le produit est obtenu selon le procédé de la préparation 1, des stades 2 à 5.
**Point de fusion : 68-70°C**

### PREPARATION 4 : 2,3,5,6-Tétrahydro-1H-cyclobuta[f]indol-6-ylméthanol

### Stade 1 : 5-Amino-1-benzocyclobutanecarboxylate de méthyle

9,74 g de 5-nitro-1-benzocyclobutanecarboxylate de méthyle sont mis à hydrogéner, à température ambiante et pression atmosphérique, en présence de Pd/C à 10 % pendant 6 h 30. Après filtration et concentration sous pression réduite, le produit attendu est isolé.

### Stade 2 : 5-[(2,2-Diméthoxyéthyl)amino]-1-benzocyclobutanecarboxylate de méthyle

Le produit est obtenu, à partir du composé du stade précédent, selon le procédé du stade 1 de la préparation 1.

### Stade 3 : 5-[N-(2,2-Diméthoxyéthyl)-N-(méthylsulfonyl)amino]-1-benzocyclobutanecarboxylate de méthyle

Le produit est obtenu, à partir du composé du stade précédent, selon le procédé du stade 2 de la préparation 1.

### Stade 4 : 1-(Méthylsulfonyl)-5,6-dihydro-1H-cyclobuta[f]indole-6-carboxylate de méthyle

Le produit est obtenu, à partir du composé du stade précédent, selon le procédé du stade 3 de la préparation 1.

### Stade 5 : [1-(Méthylsulfonyl)-5,6-dihydro-1H-cyclobuta[f]indol-6-yl]méthanol

Sur une suspension de 0,42 g d'hydrure de lithium et aluminium dans 7 ml de tétrahydrofurane, maintenue à 0°C, est ajoutée, goutte à goutte, une solution de 1,6 g du produit obtenu au stade 4 dans 20 ml de tétrahydrofurane. Après 20 minutes, le milieu réactionnel est hydrolysé par 0,3 ml d'eau, 0,23 ml de soude à 20 %, puis 1,05 ml d'eau. Après filtration des sels, le filtrat est concentré sous pression réduite pour conduire au produit attendu.

### Stade 6 : 5,6-Dihydro-1H-cyclobuta[f]indol-6-ylmethanol

Le produit est obtenu, à partir du composé du stade précédent, selon le procédé du stade 4 de la préparation 1.

### Stade 7 : 2,3,5,6-Tétrahydro-1H-cyclobuta[f]indol-6-ylméthanol

Le produit est obtenu, à partir du composé du stade précédent, selon le procédé du stade 5 de la préparation 1. Le composé est isolé après une chromatographie sur gel de silice (dichlorométhane/éthanol : 97/3).

### PREPARATION 5 : 2,3,6,7-Tétrahydro-1H-cyclobuta[g]indol-7-ylméthanol

### Stade 1 : 6-Acétyl-1-benzocyclobutanecarbonitrile

Une solution composée de 55,94 g de 6-trifluoroacétyl-1-benzocyclobutanecarbonitrile dans 600 ml de pyridine est dégazée à l'azote pendant 15 minutes ; 30,5 ml de triéthylamine, 117,44 ml de butylvinyléther, 2,25 g de 1,3-bis-(diphénylphosphino)propane et 1,02 g d'acétate de palladium sont ajoutés et le milieu réactionnel est porté au reflux pendant 2 heures. 400 ml d'acide chlorhydrique 1N sont ensuite introduits goutte à goutte en 1 heure, et après 3 heures d'agitation à température ambiante, le milieu réactionnel est extrait à l'éther. Les phases organiques sont lavées, séchées et concentrées pour conduire à un résidu qui est purifié par chromatographie sur gel de silice (dichlorométhane : 100 %) permettant d'isoler le produit attendu.
**Point de fusion : 55-59°C**

### Stade 2 : 6-Hydroxyiminoéthyl-1-benzocyclobutanecarbonitrile

10,07 g du produit du stade 1 et 6,13 g de chlorhydrate d'hydroxylamine dans 200 ml de pyridine sont agités à température ambiante pendant 19 heures. Après évaporation de la pyridine, l'huile jaune obtenue est reprise par du dichlorométhane et de l'eau. La phase organique est séparée, séchée et concentrée permettant d'isoler le produit attendu.
**Point de fusion : 108-110°C**

### Stade 3 : N-(1-Cyanobenzocyclobutan-6-yl)acétamide

A une solution de 8,9 g du produit obtenu au stade 2 dans 160 ml d'éther, refroidie à 0°C, sont ajoutés en quatre fois 9,96 g de PCl₅. Après 2 heures d'agitation à 0°C, le milieu réactionnel est ramené à température ambiante pendant 12 heures, puis versé sur un mélange eau/glace et agité pendant 20 minutes. Après décantation et extraction à l'éther, les phases organiques réunies sont séchées puis concentrées sous pression réduite pour conduire au produit attendu.
**Point de fusion : < 50°C**

### Stade 4 : 6-Amino-1-benzocyclobutanecarboxylate d'éthyle

Un courant d'HCl gazeux est introduit jusqu'à saturation d'une solution de 5,04 g du produit obtenu au stade 3 dans 400 ml d'éthanol anhydre à 0°C. Le milieu réactionnel est ensuite porté à reflux pendant 18 heures. Après concentration du solvant, le résidu est repris à l'eau glacée, basifié par une solution de carbonate de sodium et extrait au dichlorométane. La phase organique est séparée, séchée et concentrée pour obtenir le produit attendu.

### Stade 5 : 2,3,6,7-Tétrahydro-1H-cyclobuta[g]indol-7-ylméthanol

Le produit est obtenu, à partir du composé du stade précédent, selon les procédés des stades 2 à 7 de la préparation 4.

### PREPARATION 6 : 2,3,5,6-Tétrahydro-1H-cyclobuta[f]indole-5-carbonitrile

Le produit est obtenu selon le procédé de la préparation 1, des stades 1 à 5, en utilisant comme substrat au stade 1 le 4-amino-1-benzocyclobutanecarbonitrile.
**Point de fusion : 103-107°C**

### PREPARATION 7 : 2,3,5,6-Tétrahydro-1H-cyclobuta[f]indol-5-ylmethanol

Le produit est obtenu selon le procédé de la préparation 4, des stades 1 à 7, en utilisant comme substrat au stade 1 le 4-nitro-1-benzocyclobutanecarboxylate d'éthyle.

### PREPARATION 8 : Nicotinoyl azide

Sur une suspension de 12,3 g d'acide nicotinique dans 100 ml de diméthylformamide, 14,2 ml de triéthylamine sont additionnés, puis après refroidissement à 0°C, 22 ml de diphénylphosphorylazide dans 50 ml de diméthylformamide sont ajoutés. Après 2 heures d'agitation, le milieu réactionnel est coulé sur de la glace. Après extraction à l'éther, la phase organique est lavée avec une solution de NaHCO₃, séchée puis concentrée pour donner 9,68 g du produit attendu.

### PREPARATION 9 : Phényl 6-[(2-méthyl-3-pyridinyl)oxy]-3-pyridinylcarbamate

### Stade 1 : 6-[(2-Méthyl-3-pyridinyl)oxy]-3-pyridinylamine

Une solution de 14,35 g de chlorure d'étain dans 30 ml d'acide chlorhydrique concentré est ajoutée à un mélange de 5 g de 2-(2-méthylpyridi-3-yloxy)5-nitropyridine et portée à reflux pendant 1 heure. Le milieu réactionnel est refroidi et amené à pH basique par addition de soude concentré. Après filtration d'un précipité, la phase aqueuse est extraite à l'acétate d'éthyle. Après traitement usuel, le produit attendu est isolé sous la forme d'une poudre violette.
**Point de fusion : 95-100°C (M.K.)**

### Stade 2 : Phényl 6-[(2-méthyl-3-pyridinyl)oxy]-3-pyridinylcarbamate

3 ml de chloroformiate de méthyle sont ajoutés goutte à goutte sur une solution de 4,5 g du produit obtenu au stade 1, 3,3 ml de triéthylamine et 180 ml de dichlorométhane, maintenue à -20°C. Après retour à température ambiante, le milieu réactionnel est lavé au bicarbonate de sodium, séché et concentré sous pression réduite. Une chromatographie sur gel de silice du résidu (dicholrométhane/éthanol/NH₄OH : 98/2/0,29) permet d'isoler le produit attendu.

### EXEMPLE 1 : N-(3-Pyridinyl)-2,3,5,6-tétrahydro-1H-cyclobuta[f]indole-1-carboxamide

7,4 g du composé de la préparation 8 dans 40 ml de toluène sont portés à reflux pendant 2 h 30, puis refroidis à température ambiante. 0,99 g du produit de la préparation 3 dissout dans 50 ml de dichlorométhane sont alors introduits goutte à goutte. Après 18 heures d'agitation, le milieu réactionnel est filtré, et le filtrat est concentré sous pression réduite. Une chromatographie sur gel de silice (dichlorométhane/éthanol : 95/5) permet d'isoler le produit attendu qui est ensuite recristallisé de l'éthanol.
**Point de fusion : 178-180°C (M.K.)**

### EXEMPLE 2 : 6-(Hydroxyméthyl)-N-(3-pyridinyl)-2,3,5,6-tétrahydro-1H-cyclobuta [f]indole-1-carboxamide

Le produit est obtenu selon le procédé de l'exemple 1 en utilisant comme substrat le composé de la préparation 4.
**Point de fusion : 195-200°C (M.K.)**

### EXEMPLE 3 : 6-Cyano-N-(3-pyridinyl)-2,3,5,6-tétrahydro-1H-cyclobuta[f]indole-1-carboxamide

Le produit est obtenu selon le procédé de l'exemple 1 en utilisant comme substrat le composé de la préparation 1.
**Point de fusion : 203-207°C (M.K.)**

### EXEMPLE 4 : 5-Cyano-N-(3-pyridinyl)-2,3,5,6-tétrahydro-1H-cyclobuta[f]indole-1-carboxamide

Le produit est obtenu selon le procédé de l'exemple 1 en utilisant comme substrat le composé de la préparation 6.
**Point de fusion : 209-211°C (M.K.)**

### EXEMPLE 5 : 5-(Hydroxyméthyl)-N-(3-pyridinyl)-2,3,5,6-tetrahydro-1H-cyclobuta [f]indole-1-carboxamide

Le produit est obtenu selon le procédé de l'exemple 1 en utilisant comme substrat le composé de la préparation 7.
**Point de fusion : 167-173°C (M.K.)**

### EXEMPLE 6 : 7-Cyano-N-(3-pyridinyl)-1,2,6,7-tétrahydro-3H-cyclobuta[e]indole-3-carboxamide

Le produit est obtenu selon le procédé de l'exemple 1 en utilisant comme substrat le composé de la préparation 2.
**Point de fusion : 203-205°C (M.K.)**

### EXEMPLE 7 : 7-(Hydroxyméthyl)-N-(3-pyridinyl)-2,3,6,7-tétrahydro-1H-cyclobuta [g]indole-1-carboxamide

Le produit est obtenu selon le procédé de l'exemple 1 en utilisant comme substrat le composé de la préparation 5.
**Point de fusion : 216-220°C (M.K.)**

### EXEMPLE 8 : 6-[(Diméthylamino)méthyl]-6-(1-hydroxycyclohexyl)-N-(3-pyridinyl)-2,3,5,6-tétrahydro-1H-cyclobuta[f]indole-1-carboxamide

### Stade 1 : 6-(1-Hydroxycyclohexyl)-2,3,5,6-tétrahydro-1H-cyclobuta[f]indole-6-carbonitrile

4,1 g du produit obtenu à la préparation 1 sont dissous dans 215 ml de tétrahydrofurane. Le milieu réactionnel est refroidi à -80°C et 19,25 ml d'une solution 2,5 M de n-butyllithium dans l'hexane sont additionnés par l'intermédiaire d'un pousse-seringue. A la fin de l'addition, l'agitation est maintenue 20 minutes puis 6,2 ml de cyclohexanone sont coulés en 3 minutes. Après 2 heures de contact à -80°C, on laisse revenir à température ambiante et introduit 23 ml d'une solution aqueuse saturée de chlorure d'ammonium et encore 135 ml d'eau. Après décantation, la phase organique est lavée par une solution saturée de chlorure de sodium, séchée, concentrée. Le résidu obtenu est concrétisé de l'éther isopropylique, filtré pour obtenir le produit désiré et le filtrat est purifié par chromatographie sur gel de silice (CH₂Cl₂/AcOEt : 90/10) pour isoler une quantité supplémentaire de produit attendu.
**Point de fusion : 168-170°C**

### Stade 2 : 6-(1-Hydroxycyclohexyl)-1-(méthylsulfonyl)-2,3,5,6-tétrahydro-1H-cyclobuta[f]indole-6-carbonitrile

4 g du produit obtenu au stade 1 sont mis en réaction dans les conditions du stade 2 de la préparation 1.

### Stade 3 : 1-[6-(Aminométhyl)-1-(méthylsulfonyl)-2,3,5,6-tétrahydro-1H-cyclobuta[f]indol-6-yl]cyclohexanol

5,5 g du produit obtenu au stade 2 sont dissous dans 250 ml d'une solution 3,6 N de méthanol ammoniacal contenant 2 mg de nickel de Raney. Le mélange réactionnel est hydrogéné sous une pression de 30 bars à 60°C pendant 24 heures. Après filtration et évaporation du solvant, le résidu est repris par du dichlorométhane, lavé à l'eau à neutralité, séché et concentré pour isoler le produit attendu.

### Stade 4 : 1-[6-[(Diméthylamino)méthyl]-1-(méthylsulfonyl)-2,3,5,6-tétrahydro-1H-cyclobuta[f]indol-6-yl]cyclohexanol

5,37 g de l'amine obtenue au stade 3 sont dissous dans 130 ml d'acétonitrile. Dans la solution refroidie à 0°C, 2,9 g de cyanoborohydrure de sodium et 6,9 ml d'une solution de formaldéhyde à 37 % dans l'eau sont introduits en maintenant la température à 0°C. Après 20 heures de réaction à température ambiante, le milieu est hydrolysé par 210 ml d'acide chlorhydrique 1N puis agité 3 heures. Le mélange réactionnel est lavé avec 30 ml d'éther puis basifié par de la soude à 20 %. La phase aqueuse est extraite au dichlorométhane. Après séchage et évaporation, le résidu est purifié par chromatographie sur gel de silice (CH₂Cl₂/EtOH : 95/5) pour conduire au produit attendu.
**Point de fusion : 156-158°C (M.K.)**

### Stade 5 : 1-{6-[(Diméthylamino)méthyl]-2,3,5,6-tétrahydro-1H-cyclobuta[f]indol-6-yl}cyclohexanol

3,75 g du produit obtenu au stade 4 dans 100 ml de tétrahydrofurane sont ajoutés goutte à goutte sur 400 ml d'ammoniac liquide refroidi à -50°C. 0,56 g de sodium sont ajoutés par portions et l'agitation est maintenue durant 15 minutes. La réaction est stoppée par addition de 2,65 g de chlorure d'ammonium. On laisse revenir à température ambiante pour évaporer l'ammoniac puis reprend par de l'eau et extrait à l'éther. On sèche, évapore et recristallise de l'acétonitrile pour conduire au produit attendu.
**Point de fusion : 159-161°C (M.K.)**

### Stade 6 : 6-[(Diméthylamino)méthyl]-6-(1-hydroxycyclohexyl)-N-(3-pyridinyl)-2,3,5,6-tétrahydro-1H-cyclobuta[f]indole-1-carboxamide

Le produit est obtenu selon le procédé de l'exemple 1, en utilisant comme substrat le produit obtenu au stade 5.
**Point de fusion : 207-209°C**

### EXEMPLE 9 : 6-Cyano-6-(phénylsulfanyl)-N-(3-pyridinyl)-2,3,5,6-tétrahydro-1H-cyclobuta[f]indole-1-carboxamide

### Stade 1 : 6-(Phénylsulfanyl)-2,3,5,6-tétrahydro-1H-cyclobuta[f]indole 6-carbonitrile

A une solution de 2 g du produit de la préparation 1 dissout dans 40 ml de tétrahydrofurane, refroidie à -70°C sont ajoutés en 30 minutes 15 ml d'une solution 1,6 N de *n*-butyllithium dans l'hexane, puis 2,82 g de diphénylsulfure dissous dans 8 ml de tétrahydrofurane sont coulés. Le milieu réactionnel est ramené lentement à température ambiante. Après 2 heures de réaction, le milieu est versé sur 1,2 1 d'une solution saturée en chlorure d'ammonium puis extrait à l'éther. Les phases organiques réunies sont purifiées par passage acido-basique, permettant d'isoler le produit attendu.

### Stade 2 : 6-Cyano-6-(phénylsulfanyl)-N-(3-pyridinyl)-2,3,5,6-tétrahydro-1H-cyclobuta[f]indole-1-carboxamide

0,43 g du produit obtenu au stade 1 sont traités par 0,297 g du composé de la préparation 8, selon le procédé décrit à l'exemple 1, permettant d'obtenir le produit attendu.
Point de fusion : 110-113°C (M.K.)

### EXEMPLE 10 : 6-Cyano-6-cyclohéxyl-N-(3-pyridinyl)-2,3,5,6-tétrahydro-1H-cyclobuta[f]indole-1-carboxamide

### Stade 1 : 6-cyclohéxyl-2,3,5,6-tétrahydro-1H-cyclobuta[f]indole 6-carbonitrile

A une solution de 2 g du produit de la préparation 1 dissout dans 20 ml de tétrahydrofurane, refroidie à -70°C sont ajoutés en 30 minutes 15 ml d'une solution 1,6 N de *n*-butyllithium dans l'hexane. Après 30 minutes de contact, 2,17 ml de bromocyclohexane sont coulé »s en 25 minutes et le milieu réactionnel est progressivement ramené à -20°C puis versé sur une solution saturée de chlorure d'ammonium et enfin extrait à l'éther. Une purification sur silice (dichlorométhane/acétate d'éthyle : 95/5) conduit au produit attendu sous forme d'huile.

### Stade 2 : 6-Cyano-6-cyclohéxyl-N-(3-pyridinyl)-2,3,5,6-tétrahydro-1H-cyclobuta[f]indole-1-carboxamide

0,33 g du produit obtenu au stade 1 sont traités par 0,25 g du composé de la préparation 8, selon le procédé décrit à l'exemple 1, permettant d'obtenir le produit attendu.
**Point de fusion : 224-227°C**

### EXEMPLE 11 : 6-Cyclohéxyl-N-(3-pyridinyl)-2,3,5,6-tétrahydro-1H-cyclobuta[f]indole-1-carboxamide

### Stade 1 : 6-Cyclohéxyl-2,3,5,6-tétrahydro-1H-cyclobuta[f]indole

Une solution de 0,5 g de produit obtenu au stade 1 de l'exemple 10 dans 10 ml de tétrahydrofurane anhydre et 0,11 ml d'éthanol absolu sont coulés sur 20 ml d'ammoniac liquide à une température de -78°C, puis 0,15 g de sodium sont introduits par portions. Après 30 minutes de contact à cette température le milieu réactionnel est traité par 0,83 g de chlorure d'ammonium. Après évaporation de l'ammoniac, le résidu est repris par une solution saturée de chlorure d'ammonium, extrait à l'éther et les phases éthérées sont lavées, séchées et concentrées pour conduire au produit attendu.

### Stade 2 : 6-Cyclohéxyl-N-(3-pyridinyl)-2,3,5,6-tétrahydro-1H-cyclobuta[f]indole-1-carboxamide

0,43 g du produit obtenu au stade 1 sont traités par 0,36 g du composé de la préparation 8, selon le procédé décrit à l'exemple 1, permettant d'obtenir le produit attendu.
**Point de fusion : 187-190°C (M.K.)**

### EXEMPLE 12 : 6-Cyano-N-{6-[(2-méthyl-3-pyridinyl)oxy]-3-pyridinyl}-6-(phénylsulfanyl)-2,3,5,6-tétrahydro-1H-cyclobuta[f]indole-1-carboxamide

Un mélange composé de 0,5 g du produit obtenu u stade 1 de l'exemple 9, 0,6 g du produit de la préparation 9, 0,16 ml de triéthylamine et 16 ml de diméthylformamide est porté à 100°C pendant 1 heure. Après évaporation de la diméthylformamide, le résidu est repris par 100 ml de dichlorométhane, lavé avec de la soude à 10%, à l'eau, puis séché et concentré sous pression réduite, pour conduire après purification sur silice (dichlorométhane/ éthanol/NH₄OH / 98/2/0,4) et recristallisation de l'acétonitrile, au produit attendu

### ETUDE PHARMACOLOGIQUE DES COMPOSES DE L'INVENTION

### EXEMPLE 13 : Test de conflit de Vogel

Le test s'effectue sur des rats mâles Wistar (IFFA-CREDO) de poids 230-250 g, stabulés en animalerie par groupes de 4 sur sciure, dans des cages avec boisson et nourriture à volonté pendant 5 jours avant leur utilisation et soumis aux conditions suivantes : température (21 ± 1°C), humidité (60 ± 5 %) et cycle diurne de 12 heures (7:00 à 19:00 h). Le lundi suivant leur arrivée, les animaux sont transférés dans la pièce d'expérimentation où ils séjourneront jusqu'au vendredi, jour de Test. Pendant 4 jours, du lundi au jeudi inclus, les animaux n'ont alors qu'une seule heure par jour d'accès à la boisson (de 9:00 à 10:00 h).
La veille du test, à partir de 15:00 h, les animaux sont isolés dans des cages sur grille, sans boisson ni nourriture.
Le test se déroule dans une cage plastique transparente située dans une enceinte insonorisée et ventilée. La cage comporte un plancher en acier chromé. L'embout métallique du flacon contenant la boisson pénètre dans la cage à une hauteur de 6 cm au-dessus du plancher métallique. Le plancher et l'embout du flacon sont reliés par des câbles électriques à un appareil qui assure l'enregistrement des lèchements de l'animal et contrôle l'administration des chocs électriques. L'appareil est réglé de telle sorte que l'animal reçoit un choc électrique (entre embout et plancher métallique) tous les 20 lèchements de l'embout.
Le jour du test, l'animal reçoit une injection (s.c.) de sérum physiologique (Témoin) ou de produit à tester, 30 minutes avant d'être placé dans la cage Test. La session débute dès que l'animal a effectué 20 lèchement et a reçu un premier choc électrique (durée 0,5 sec, intensité 0,300 mA). Pendant 3 minutes, l'animal reçoit un choc électrique chaque fois qu'il effectue 20 lèchements.
Les résultats sont les nombres de lèchements et de chocs reçus par l'animal pendant les 3 minutes du Test.
Les nombres de lèchements et de chocs reçus par les animaux traités sont comparés à ceux respectifs des animaux témoins, par analyse de variance, suivie d'un test de Dunnett avec P < 0,05. Un produit anxiolytique augmente le nombre de lèchements et de chocs reçus par l'animal, par rapport aux Témoins. A titre indicatif, le nombre moyen de lèchements non punis chez des animaux assoiffés est de 674,9 ± 44,5 (N = 7) en 3 minutes.
L'efficacité d'un produit est exprimée par la dose minimale efficace (M.E.D.), c'est-à-dire la plus faible dose produisant une différence significative par rapport aux Témoins. Cette dose est de 2,5 mg/kg, s.c. pour le produit de l'exemple 1.

| ***Résultats du produit de l'exemple n°1*** | | |
|---|---|---|
| ***Doses mg*/*kg, s.c.*** | ***Lèchements punis (1 choc*/*20 lèchements)*** | ***N*** |
| 0 | 142,7 ± 31,9 | 10 |
| 0,63 | 197,5 ± 59,5 | 8 |
| 2,5 | 560,1 ± 67,0* | 8 |
| 10,0 | 499,3 ± 95,3* | 7 |

| | | |
|---|---|---|
| * P < 0,05 | | |

### EXEMPLE 14 : Test d'enfouissement des billes chez la souris

Ce test permet d'évaluer la capacité d'agents pharmacologiques à inhiber le comportement spontané d'enfouissement de billes chez la souris, cette inhibition étant prédictive d'actions antidépressive et/ou anti-impulsive. Des souris mâles de souche NMRI (Iffa-Credo, L'Arbresle, France), pesant 20-25 g le jour de l'expérience, sont placées individuellement dans des boîtes en Macrolon (30 x 18 x 19 cm) contenant 5 cm de sciure et recouvertes par une plaque en plexiglass perforée. Vingt-quatre billes en verre "oeil de chat" sont réparties régulièrement sur la sciure à la périphérie de la boîte. Au terme de 30 minutes d'exploration libre, les animaux sont retirés de la boîte et le nombre de billes enfouies est comptabilisé.
A titre d'exemple, la MED (dose minimale efficace) pour le produit de l'exemple 1 est de 2,5 mg/kg s.c.

### EXEMPLE 15 : Composition pharmaceutique : comprimés

| *Formule de préparation pour 1000 comprimés dosés à 5 mg* | |
|---|---|
| Composé de l'exemple 1 | 5 g |
| Hydroxypropylméthylcellulose | 5 g |
| Amidon de blé | 10 g |
| Lactose | 100 g |
| Stéarate de Magnésium | 2 g |

## Revendications

1. Composés de formule **(I)** : 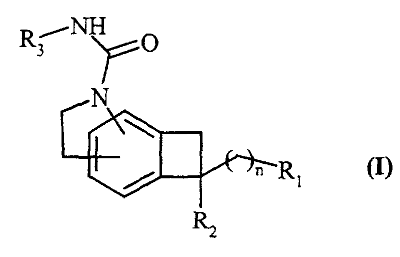 dans laquelle :
**n** représente un entier compris entre 0 et 6,
**R**_{**1**} représente un groupement choisi parmi hydrogène, hydroxy, cyano, alkoxy (C₁-C₆) linéaire ou ramifié, alkoxycarbonyle (C₁-C₆) linéaire ou ramifié, carboxy, aminocarbonyle (la partie amino étant éventuellement substituée par un ou deux groupements, identiques ou différents, choisis parmi alkyle (C₁-C₆) linéaire ou ramifié, aryle et arylalkyle (C₁-C₆) linéaire ou ramifié), et NR₄R₅ dans lequel R₄ et R₅, identiques ou différents, représentent un groupement choisi parmi alkyle (C₁-C₆) linéaire ou ramifié, aryle, arylalkyle (C₁-C₆) linéaire ou ramifié, hétéroaryle, hétéroarylalkyle (C₁-C₆) linéaire ou ramifié, cycloalkyle, cycloalkylalkyle (C₁-C₆) linéaire ou ramifié, alkényle (C₂-C₆) linéaire ou ramifié, et alkynyle (C₂-C₆) linéaire ou ramifié,
**R**_{**2**} représente un groupement choisi parmi atome d'hydrogène, groupement alkyle (C₁-C₆) linéaire ou ramifié, hydroxyméthyle, groupement de formule 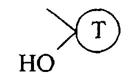 et -U-V-W dans lesquels :
* T représente un groupement cycloalkyle (C₃-C₁₂) monocyclique ou polycyclique, dont l'un des atomes de carbone du cycloalkyle peut éventuellement être remplacé par un groupement choisi parmi atome d'oxygène, atome de sélénium, groupement de formule S(O)ₚ dans laquelle p représente un entier compris entre 0 et 2 inclus, et groupement de formule SiR₆R₇ dans laquelle R₆ et R₇, identiques ou différents, représentent un groupement alkyle (C₁-C₆) linéaire ou ramifié,
* U représente une liaison ou un groupement méthylène,
* V représente une liaison, un atome d'oxygène ou un groupement S(O)_{q} dans lequel q est un entier compris entre 0 et 2 inclus,
* W représente un groupement choisi parmi aryle, arylalkyle (C₁-C₆) linéaire ou ramifié, cycloalkyle, et cycloalkylalkyle (C₁-C₆) linéaire ou ramifié,
**R**_{**3**} représente un groupement choisi parmi atome d'hydrogène, groupement alkyle (C₁-C₆) linéaire ou ramifié, aryle, et hétéroaryle,
leurs isomères ainsi que leurs sels d'addition à un acide ou à une base pharmaceutiquement acceptable,
étant entendu que :
• par groupement aryle, on comprend un groupement choisi parmi phényle, biphényle, naphtyle, dihydronaphtyle, tétrahydronaphtyle, indanyle, indényle, et benzocyclobutyle, chacun de ces groupements étant éventuellement substitué par un ou plusieurs groupements, identiques ou différents, choisis parmi atomes d'halogène, groupements alkyle (C₁-C₆) linéaire ou ramifié, hydroxy, alkoxy (C₁-C₆) linéaire ou ramifié, nitro, cyano, trihalogénoalkyle (C₁-C₆) linéaire ou ramifié, amino, monoalkylamino, dialkylamino (C₁-C₆) linéaire ou ramifié, trihalogénoalkoxy (C₁-C₆) linéaire ou ramifié, aminoalkyl(C₁-C₆)aminocarbonyle (les atomes d'azote de chacune des parties amino étant éventuellement substitués par des groupements alkyle (C₁-C₆) linéaires ou ramifiés, identiques ou différents), pyridinyle, pyridinyloxy, pyridinyloxyméthyle, ces trois derniers groupements étant éventuellement substitués par un groupement alkyle (C₁-C₆) linéaire ou ramifié,
• par groupement hétéroaryle, on comprend un système monocyclique aromatique ou bicyclique dont l'un des cycles est aromatique, l'autre cycle étant aromatique ou partiellement hydrogéné, de 5 à 12 chaînons, contenant un, deux ou trois hétéroatomes, identiques ou différents, choisis parmi oxygène, azote et soufre, chacun de ces groupements étant éventuellement substitué par un ou plusieurs groupements, identiques ou différents, choisis parmi les substituants décrits pour le groupement aryle précédemment défini,
• et par groupement cycloalkyle, on comprend un système mono- ou polycyclique, de 3 à 12 chaînons, contenant éventuellement une ou plusieurs insaturations, celles-ci ne conférant pas de caractère aromatique audit système cyclique.

2. Composés de formule (I) selon la revendication 1 **caractérisés en ce que** R₂ représente un atome d'hydrogène, leurs isomères ainsi que leurs sels d'addition à un acide ou à une base pharmaceutiquement acceptable.

3. Composés de formule (I) selon la revendication 1 **caractérisés en ce que** R₂ représente un groupement de formule 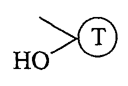 dans laquelle T est tel que défini dans la formule (I), n est égal à 1, et R₁ représente un groupement cyano ou un groupement amino substitué par un ou deux groupements, identiques ou différents, choisis parmi alkyle (C₁-C₆) linéaire ou ramifié, et arylalkyle (C₁-C₆) linéaire ou ramifié, leurs isomères ainsi que leurs sels d'addition à un acide ou à une base pharmaceutiquement acceptable.

4. Composés de formule (I) selon la revendication 1 **caractérisés en ce que** n est égal à 0, R₁ représente un atome d'hydrogène ou un groupement cyano, et R₂ représente un atome d'hydrogène, leurs isomères ainsi que leurs sels d'addition à un acide ou à une base pharmaceutiquement acceptable.

5. Composés de formule (I) selon la revendication 1 **caractérisés en ce que** n est égal à 0, R₁ représente un atome d'hydrogène ou un groupement cyano, et R₂ représente un groupement de formule -U-V-W dans laquelle U représente une liaison simple, V représente un groupement de formule S(O)ₚ avec p tel que défini dans la formule (I) et W représente un groupement aryle, leurs isomères ainsi que leurs sels d'addition à un acide ou à une base pharmaceutiquement acceptable.

6. Composés de formule (I) selon la revendication 1 **caractérisés en ce que** R₃ représente un groupement hétéroaryle, leurs isomères ainsi que leurs sels d'addition à un acide ou à une base pharmaceutiquement acceptable.

7. Composés de formule (I) selon la revendication 1 **caractérisés en ce que** R₃ représente un groupement pyridinyle, leurs isomères ainsi que leurs sels d'addition à un acide ou à une base pharmaceutiquement acceptable.

8. Composés de formule (I) selon la revendication 1 qui sont le :
- *N*-(3-pyridinyl)-2,3,5,6-tétrahydro-1*H*-cyclobuta[*f*]indole-1-carboxamide,
- 5-cyano-N-(3-pyridinyl)-2,3,5,6-tétrahydro-1*H*-cyclobuta[*f*]indole-1-carboxamide,
- 6-cyano-N-(3-pyridinyl)-2,3,5,6-tétrahydro-1*H*-cyclobuta[*f*]indole-1-carboxamide,
- 6-(hydroxyméthyl)-N-(3-pyridinyl)-2,3,5,6-tétrahydro-1*H*-cyclobuta[*f*]indole-1-carboxamide,
- 5-(hydroxyméthyl)-N-(3-pyridinyl)-2,3,5,6-tétrahydro-1*H*-cyclobuta[*f*]indole-1-carboxamide,
- 7-cyano-N-(3-pyridinyl)-1,2,6,7-tétrahydro-3*H*-cyclobuta[*e*]indole-3-carboxamide,
- 7-(hydroxyméthyl)-N-(3-pyridinyl)-2,3,6,7-tétrahydro-1*H*-cyclobuta[*g*]indole-1-carboxamide,
- 6-[(diméthylamino)méthyl]-6-(1-hydroxycyclohexyl)-N-(3-pyridinyl)-2,3,5,6-tétrahydro-1*H*-cyclobuta[*f*]indole-1-carboxamide,
- 6-cyano-6-(phénylsulfanyl)-N-(3-pyridinyl)-2,3,5,6-tétrahydro-1*H*-cyclobuta[f]indole-1-carboxamide,
- 6-cyano-6-cyclohéxyl-N-(3-pyridinyl)-2,3,5,6-tétrahydro-1*H*-cyclobuta[f]indole-1-carboxamide,
- 6-cyclohéxyl-N-(3-pyridinyl)-2,3,5,6-tétrahydro-1*H*-cyclobuta[f]indole-1-carboxamide,
- 6-cyano-N-{6-[(2-méthyl-3-pyridinyl)oxy]-3-pyridinyl}-6-(phénylsulfanyl)-2,3,5,6-tétrahydro-1*H*-cyclobuta[f]indole-1-carboxamide,
leurs isomères ainsi que leurs sels d'addition à un acide ou à une base pharmaceutiquement acceptable.

9. Procédé de préparation des composés de formule (I) **caractérisé en ce qu'**on utilise comme produit de départ un composé de formule **(II)** : 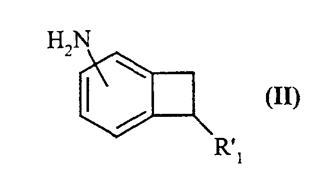 dans laquelle R'₁ représente un groupement choisi parmi atome d'hydrogène, groupement cyano, hydroxyméthylène, carboxy, et alkoxycarbonyle (C₁-C₆) linéaire ou ramifié,
composé de formule (II) qui est mis à réagir dans des conditions d'amination réductrice avec un composé de formule **(III)** :
(AO)₂CHCHO **(III)**
dans laquelle A représente un groupement alkyle (C₁-C₆) linéaire ou ramifié, pour conduire aux composés de formule **(IV)** : 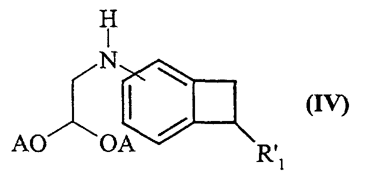 dans laquelle A et R'₁ sont tels que définis précédemment,
composés de formule (IV) qui sont traités par un composé de formule **(V) :**
ClSO₂E **(V)**
dans laquelle E représente un groupement alkyle (C₁-C₄) linéaire ou ramifié, phényle ou p-toluyle, pour conduire aux composés de formule **(VI)** : 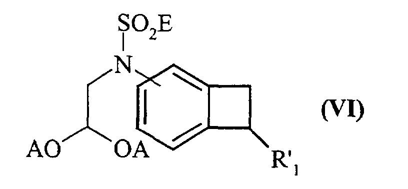 dans laquelle A, E et R'₁ sont tels que définis précédemment,
composés de formule (VI) qui sont cyclisés dans des conditions acides, pour conduire aux composés de formule **(VII)** : 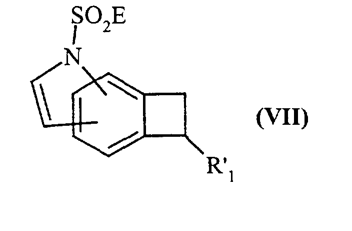 dans laquelle E et R'₁ sont tels que définis précédemment,
composés de formule (VII) qui sont traités soit par un hydroxyde de métal alcalin dans un solvant alcoolique, soit par le sodium dans l'ammoniaque liquide pour conduire aux composés de formule **(VIII)** : 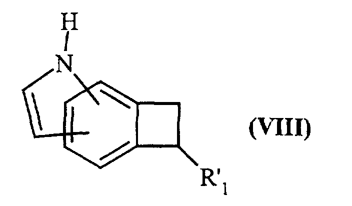 dans laquelle R'₁ est tel que défini précédemment,
composés de formule (VIII) qui sont ensuite réduits selon des conditions classiques de la synthèse organique, pour conduire aux composés de formule **(IX) :** 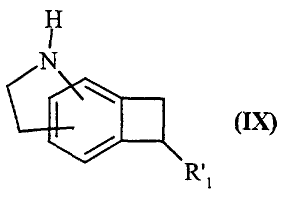 dans laquelle R'₁ est tel que défini précédemment,
composés de formule (IX) qui sont traités par un isocyanate de formule **(X)** :
R₃-N=C=O **(X)**
dans laquelle R₃ est tel que défini dans la formule (I),
pour conduire aux composés de formule **(I/a)**, cas particulier des composés de formule (I) : 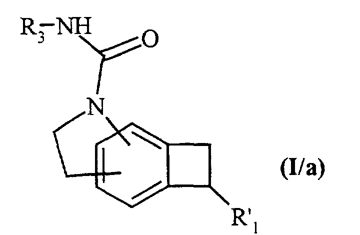 dans laquelle R'₁ et R₃ sont tels que définis précédemment,
ou composés de formule **(IX)**, dans le cas particulier où R'₁ représente un groupement cyano, que l'on traite :
* *soit* par une cétone de formule **(XI)** : 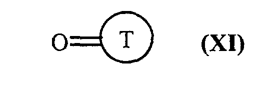 dans laquelle T a les mêmes significations que dans la formule (I), pour conduire aux composés de formule **(XII)** : 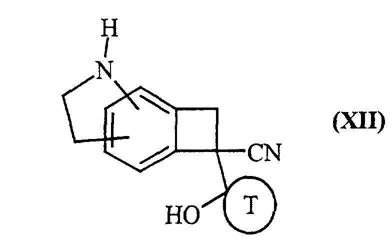 dans laquelle T est tel que défini précédemment,
composés de formule (XII) qui sont ensuite :
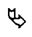 **soit** traités par un isocyanate de formule (X) telle que décrite précédemment, pour conduire aux composés de formule **(I/b)**, cas particulier des composés de formule (I), 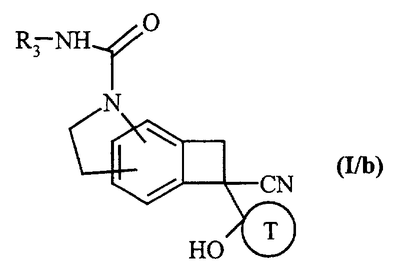 dans laquelle T et R₃ sont tels que définis précédemment,
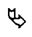 **soit,** après protection de l'amine du groupe indoline, réduits selon les méthodes classiques de la synthèse organique, pour conduire aux composés de formule **(XIII)** : 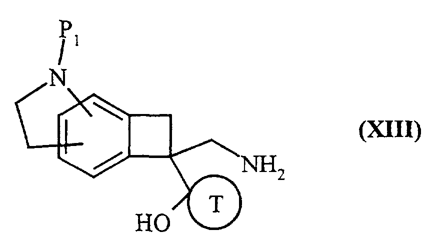 dans laquelle T est tel que défini précédemment et P₁ est un groupement protecteur classique,
composés de formule (XIII) dont la fonction amine primaire est ensuite substituée et transformée en fonction amine secondaire puis tertiaire, en utilisant des méthodes usuelles de la chimie organique, pour conduire aux composés de formule **(XIV) :** 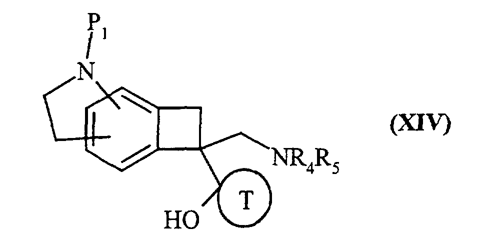 dans laquelle R₄ et R₅ ont les mêmes significations que dans la formule (I) et, T et P₁ sont tels que définis précédemment,
composés de formule (XIV) qui, après déprotection de l'atome d'azote du noyau indoline, sont traités par un composé de formule (X) telle que décrite précédemment, pour conduire aux composés de formule **(I/c)**, cas particulier des composés de formule (I) : 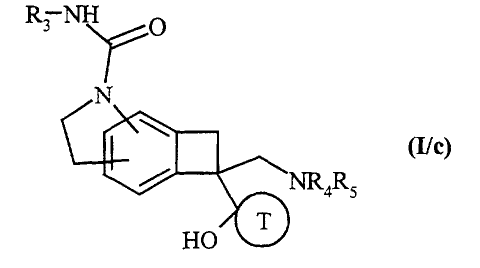 dans laquelle T, R₄, R₅ et R₃ sont tels que définis précédemment,
* *soit* par une base forte ou un alcoolate de métal alcalin, en présence d'un composé de formule **(XV)** :
W₁ - X **(XV)**
dans laquelle W₁ représente un groupement alkyle (C₁-C₆) linéaire ou ramifié, arylalkyle (C₁-C₆) linéaire ou ramifié, cycloalkyle, ou cycloalkylalkyle (C₁-C₆) linéaire ou ramifié, et X représente un groupe partant tel qu'un atome d'halogène, un groupement trifluorométhylsulfonate, mésylate ou tosylate,
pour conduire aux composés de formule **(XVI)** : 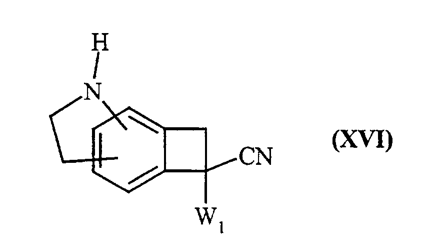 dans laquelle W₁ est tel que défini précédemment,
composés de formule (XVI) qui sont :
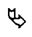 **soit** traités par un composé de formule (X) tel que décrit précédemment, pour conduire aux composés de formule **(I/d)**, cas particulier des composés de formule (I) : 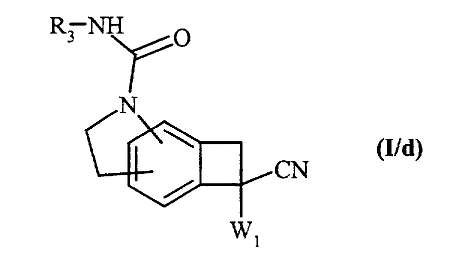 dans laquelle W₁ et R₃ sont tels que définis précédemment,
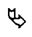 **soit** transformé, après protection de l'atome d'azote du noyau indoline, comme les composés de formule (XII) en amine primaire, secondaire puis tertiaire, pour conduire après déprotection et traitement en présence d'un composé de formule (X) telle que décrite précédemment, aux composés de formule **(I/e),** cas particulier des composés de formule (I) : 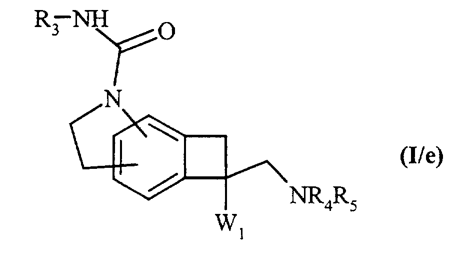 dans laquelle W₁, R₃, R₄ et R₅ sont tels que définis précédemment,
* *soit* par du dibrome dans un solvant organique chloré pour conduire aux composés de formule **(XVII)** : 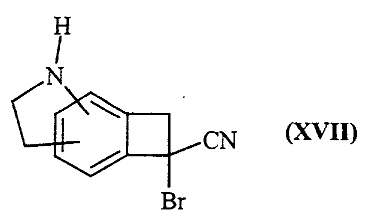 composés de formule (XVII) qui sont mis à réagir avec un composé de formule **(XVIII)** :
W - V₁ - H **(XVIII)**
dans laquelle W a la même signification que dans la formule (I) et V₁ représente un atome d'oxygène ou un atome de soufre,
pour conduire aux composés de formule **(XIX) :** 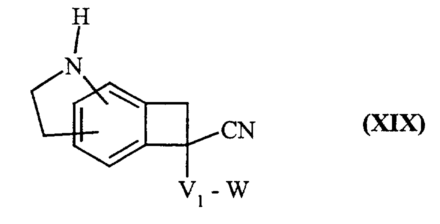 dans laquelle V₁ et W sont tels que définis précédemment,
composés de formule (XIX) qui sont :
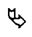 **soit** traités par un composé de formule (X) tel que décrit précédemment, pour conduire aux composés de formule **(I/f)**, cas particulier des composés de formule (I) : 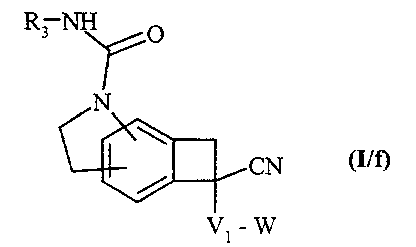 dans laquelle R₃, V₁ et W sont tels que définis précédemment,
composés de formule (I/f), dans le cas particulier où V₁ représente un atome de soufre, qui peuvent être soumis à une oxydation dans des conditions classiques de la synthèse organique, pour conduire aux composés de formule **(I/g)**, cas particulier des composés de formule (I) : 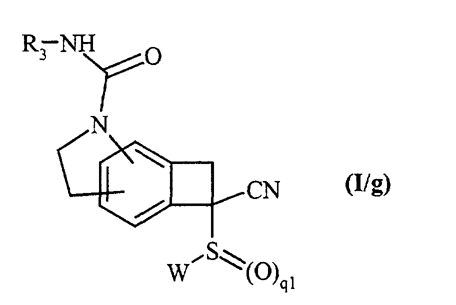 dans laquelle R₃ et W sont tels que définis dans la formule (I) et q₁ est un entier compris entre 1 et 2 inclus,
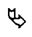 **soit** protégés puis transformés, par la même séquence de réactions que les composés de formule (XII), en amine primaire, secondaire et tertiaire, pour conduire après déprotection et traitement par un composé de formule (X), tel que décrit précédemment, aux composés de formule **(I/h)**, cas particulier des composés de formule (I) : 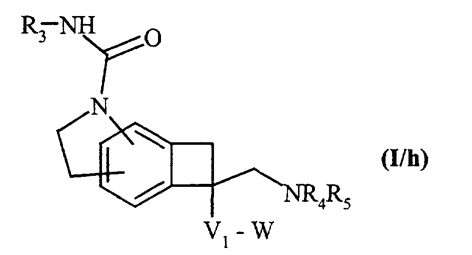 dans laquelle V₁, W, R₃, R₄ et R₅ sont tels que définis précédemment,
composés de formule (I/h) dans le cas particulier où V₁ représente un atome de soufre, qui peuvent être soumis à une oxydation dans des conditions classiques de la synthèse organique, pour conduire aux composés de formule **(I/i)**, cas particulier des composés de formule (I) : 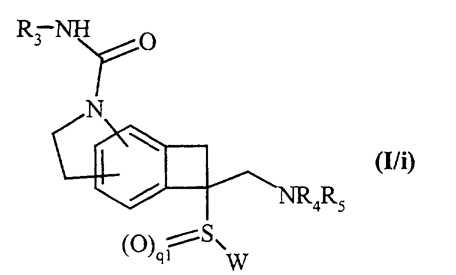 dans laquelle W, R₃, R₄, R₅ et q₁ sont tels que définis précédemment,
* *soit* par un hydrure alcalin dans la diméthylformamide en présence de formaldéhyde, pour conduire aux composés de formule **(XX)** : 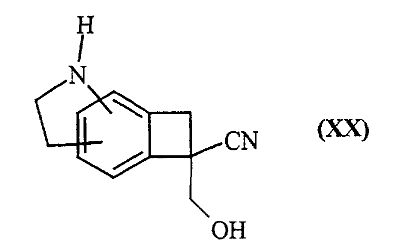 composés de formule (XX) qui sont :
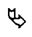 **soit** traités par un composé de formule (X) tel que décrit précédemment, pour conduire aux composés de formule **(I/j)**, cas particulier des composés de formule (I) : 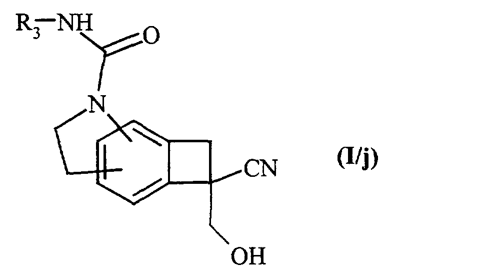 dans laquelle R₃ est tel que défini dans la formule (I),
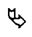 **soit** protégés au niveau de l'atome d'azote du noyau indoline, puis traités selon les conditions de la réaction de Mitsunobu par un composé de formule **(XXI)** :
W - OH **(XXI)**
dans laquelle W est tel que défini dans la formule (I),
pour conduire, après déprotection de l'atome d'azote du noyau indole, aux composés de formule **(XXII)** : 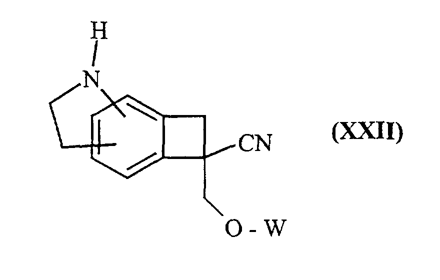 dans laquelle W est tel que défini précédemment,
composés de formule (XXII) qui sont :
◆ ***soit*** traités par un composé de formule (X) tel que décrit précédemment, pour conduire aux composés de formule **(I/k)**, cas particulier des composés de formule (I) : 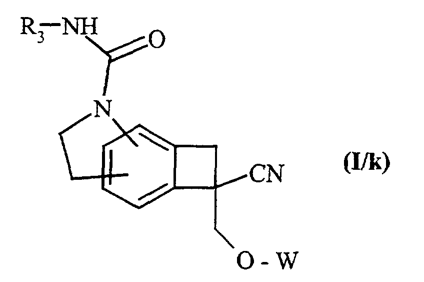 dans laquelle R₃ et W sont tels que définis précédemment,
◆ ***soit*** protégés puis transformés, par la même séquence de réaction que les composés de formule (XII), en amine primaire, secondaire et tertiaire, pour conduire après déprotection et traitement par un composé de formule (X), tel que décrit précédemment, aux composés de formule **(I/l)**, cas particulier des composés de formule (I) : 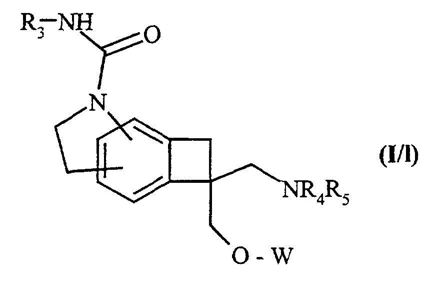 dans laquelle R₃, R₄, R₅ et W sont tels que définis précédemment,
les composés (I/a) à (I/l) forment l'ensemble des composés de l'invention, que l'on purifie le cas échéant, selon une technique classique de purification, qui peuvent, si on le désire, être séparés en leurs différents isomères selon une technique classique de séparation, et que l'on transforme, le cas échéant, en leurs sels d'addition à un acide ou à une base pharmaceutiquement acceptable.

10. Compositions pharmaceutiques contenant comme principe actif au moins un composé de formule (I) selon l'une quelconque des revendications 1 à 9, seul ou en combinaison avec un ou plusieurs excipients ou véhicules inertes, non toxiques, pharmaceutiquement acceptables.

11. Compositions pharmaceutiques selon la revendication 10 contenant au moins un principe actif selon l'une quelconque des revendications 1 à 9 utiles, en tant que médicament, dans le traitement de l'anxiété, des attaques de paniques, des troubles obsessionnels compulsifs, des phobies, des troubles impulsifs, de l'abus de drogue, des troubles de la cognition, des psychoses, des dépressions, et des troubles de l'humeur.

## Patentansprüche

1. Verbindungen der Formel **(I)**: 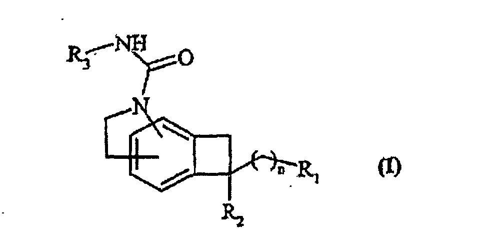 in der:
**n** eine ganze Zahl zwischen 0 und 6 bedeutet,
**R**_{**1**} eine Gruppe bedeutet ausgewählt aus Wasserstoff, Hydroxy, Cyano, geradkettigem oder verzweigtem (C₁-C₆)-Alkoxy, geradkettigem oder verzweigtem (C₁-C₆)-Alkoxycarbonyl, Carboxy, Aminocarbonyl (wobei der Aminorest gegebenenfalls durch eine oder zwei gleichartige oder verschiedenartige Gruppen ausgewählt aus geradkettigem oder verzweigtem (C₁-C₆)-Alkyl, Aryl und geradkettigem oder verzweigtem Aryl-(C₁-C₆)-alkyl substituiert ist) und NR₄R₅, worin R₄ und R₅, die gleichartig oder verschieden sind, eine Gruppe bedeuten ausgewählt aus geradkettigem oder verzweigtem (C₁-C₆)-Alkyl, Aryl, geradkettigem oder verzweigtem Aryl-(C₁-C₆)-alkyl, Heteroaryl, geradkettigem oder verzweigtem Heteroaryl-(C₁-C₆)-alkyl, Cycloalkyl, geradkettigem oder verzweigtem Cycloalkyl-(C₁-C₆)-alkyl, geradkettigem oder verzweigtem (C₂-C₆)-Alkenyl und geradkettigem oder verzweigtem (C₂-C₆)-Alkinyl,
**R**_{**2**} eine Gruppe bedeutet ausgewählt aus dem Wasserstoffatom, geradkettigen oder verzweigten (C₁-C₆)-Alkylgruppen, der Hydroxymethylgruppe und Gruppen der Formeln 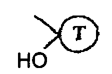 und -U-V-W,
in denen:
* T eine monocyclische oder polycyclische (C₃-C₁₂)-Cycloalkylgruppe bedeutet, bei der eines der Kohlenstoffatome des Cycloalkylrestes gegebenenfalls durch eine Gruppe ersetzt sein kann, ausgewählt aus dem Sauerstoffatom, dem Seleniumatom, einer Gruppe der Formel S(O)ₚ, in der p eine ganze Zahl mit einem Wert zwischen 0 und 2 einschließlich darstellt, und einer Gruppe der Formel SiR₆R₇, worin R₆ und R₇, die gleichartig oder verschieden sind, eine geradkettige oder verzweigte (C₁-C₆)-Alkylgruppe darstellen,
* U eine Bindung oder eine Methylengruppe bedeutet,
* V eine Bindung, ein Sauerstoffatom oder eine Gruppe S(O)_{q} darstellt, in der q eine ganze Zahl zwischen 0 und 2 einschließlich bedeutet,
* W eine Gruppe bedeutet ausgewählt aus Aryl, geradkettigem oder verzweigtem Aryl-(C₁-C₆)-alkyl, Cycloalkyl und geradkettigem oder verzweigtem Cycloalkyl-(C₁-C₆)-alkyl, und
**R**_{**3**} eine Gruppe bedeutet ausgewählt aus dem Wasserstoffatom, geradkettigen oder verzweigten (C₁-C₆)-Alkylgruppen, Arylgruppen und Heteroarylgruppen,
deren Isomere sowie deren Additionssalze mit einer pharmazeutisch annehmbaren Säure oder Base,
mit der Maßgabe, daß man:
• unter einer Arylgruppe eine Gruppe versteht ausgewählt aus Phenyl, Biphenyl, Naphthyl, Dihydronaphthyl, Tetrahydronaphthyl, Indanyl, Idenyl und Benzocyclobutyl, wobei jede dieser Gruppen gegebenenfalls durch eine oder mehrere, gleichartige oder verschiedenartige Gruppen substituiert ist, ausgewählt aus Halogenatomen, geradkettigen oder verzweigten (C₁-C₆)-Alkylgruppen, Hydroxygruppen, geradkettigen oder verzweigten (C₁-C₆)-Alkoxygruppen, Nitrogruppen, Cyanogruppen, geradkettigen oder verzweigten (C₁-C₆)-Trihalogenalkylgruppen, Aminogruppen, Monoalkylaminogruppen, geradkettigen oder verzweigten (C₁-C₆)-Dialkylaminogruppen, geradkettigen oder verzweigten (C₁-C₆)-Trihalogenalkoxygruppen, (C₁-C₆)-Aminoalkyl-aminocarbonylgruppen (wobei die Stickstoffatome jedes der Aminoreste gegebenenfalls durch gleichartige oder verschiedenartige, geradkettige oder verzweigte (C₁-C₆)-Alkylgruppen substituiert ist), Pyridinylgruppen, Pyridinyloxygruppen, Pyridinyloxymethylgruppen, wobei diese drei letzteren Gruppen gegebenenfalls durch eine geradkettige oder verzweigte (C₁-C₆)-Alkylgruppe substituiert sind,
• unter einer Heteroarylgruppe ein monocyclisches aromatisches System oder ein bicyclisches System, bei dem einer der Ringe aromatisch ist und der andere aromatisch oder teilweise hydriert ist, mit 5 bis 12 Kettengliedern, die ein, zwei oder drei gleichartige oder verschiedenartige Heteroatome ausgewählt aus Sauerstoff, Stickstoff und Schwefel enthalten, versteht, wobei jede dieser Gruppen gegebenenfalls durch eine oder mehrere gleichartige oder verschiedenartige Gruppen substituiert ist, ausgewählt aus den oben für die Arylgruppe definierten Substituenten,
• und unter einer Cycloalkylgruppe ein mono- oder polycyclisches System mit 3 bis 12 Kettengliedern, welche gegebenenfalls eine oder mehrere Unsättigungen aufweist, die dem cyclischen System jedoch keinen aromatischen Charakter verleihen, versteht.

2. Verbindungen der Formel (I) nach Anspruch 1, **dadurch gekennzeichnet, daß** R₂ ein Wasserstoffatom bedeutet, deren Isomere sowie deren Additionssalze mit einer pharmazeutisch annehmbaren Säure oder Base.

3. Verbindungen der Formel (I) nach Anspruch 1, **dadurch gekennzeichnet, daß** R₂ eine Gruppe der Formel 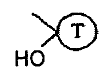 bedeutet, in der T die bezüglich der Formel (I) angegebenen Bedeutungen besitzt, n den Wert 1 besitzt und R₁ eine Cyanogruppe oder eine Aminogruppe, die durch eine oder zwei gleichartige oder verschiedenartige Gruppen ausgewählt aus geradkettigem oder verzweigtem (C₁-C₆)-Alkyl und geradkettigem oder verzweigtem Aryl-(C₁-C₆)-alkyl substituiert ist, bedeutet, deren Isomere sowie deren Additionssalze mit einer pharmazeutisch annehmbaren Säure oder Base.

4. Verbindungen der Formel (I) nach Anspruch 1, **dadurch gekennzeichnet, daß** n den Wert 0 besitzt, R₁ ein Wasserstoffatom oder eine Cyanogruppe und R₂ ein Wasserstoffatom bedeuten, deren Isomere sowie deren Additionssalze mit einer pharmazeutisch annehmbaren Säure oder Base.

5. Verbindungen der Formel (I) nach Anspruch 1, **dadurch gekennzeichnet, daß** n den Wert 0 besitzt, R₁ ein Wasserstoffatom oder eine Cyanogruppe und R₂ eine Gruppe der Formel -U-V-W bedeuten, worin U eine Einfachbindung, V eine Gruppe der Formel S(O)ₚ, worin p die bezüglich der Formel (I) angegebenen Bedeutungen besitzt, und W eine Arylgruppe bedeuten, deren Isomere sowie deren Additionssalze mit einer pharmazeutisch annehmbaren Säure oder Base.

6. Verbindungen der Formel (I) nach Anspruch 1, **dadurch gekennzeichnet, daß** R₃ eine Heteroarylgruppe bedeutet, deren Isomere sowie deren Additionssalze mit einer pharmazeutisch annehmbaren Säure oder Base.

7. Verbindungen der Formel (I) nach Anspruch 1, **dadurch gekennzeichnet, daß** R₃ eine Pyridinylgruppe bedeutet, deren Isomere sowie deren Additionssalze mit einer pharmazeutisch annehmbaren Säure oder Base.

8. Verbindungen der Formel (I) nach Anspruch 1, nämlich:
- *N*-(3-Pyridinyl)-2,3,5,6-tetrahydro-1*H*-cyclobuta[*f*]indol-1-carboxamid,
- 5-Cyano-N-(3-pyridinyl)-2,3,5,6-tetrahydro-1*H*-cyclobuta[*f*]indol-1-carboxamid,
- 6-Cyano-N-(3-pyridinyl)-2,3,5,6-tetrahydro-1*H*-cyclobuta[*f*]indol-1-carboxamid,
- 6-(Hydroxymethyl)-N-(3-pyridinyl)-2,3,5,6-tetrahydro-1*H*-cyclobuta[*f*]indol-1-carboxamid,
- 5-Hydroxymethyl)-N-(3-pyridinyl)-2,3,5,6-tetrahydro-1*H*-cyclobuta[*f*]indol-1-carboxamid,
- 7-Cyano-N-(3-pyridinyl)-1,2,6,7-tetrahydro-3*H*-cyclobuta[*e*]indol-3-carboxamid,
- 7-(Hydroxymethyl)-N-(3-pyridinyl)-2,3,6,7-tetrahydro-1*H*-cyclobuta[*g*]indol-1-carboxamid,
- 6-[(Dimethylamino)-methyl]-6-(1-hydroxycyclohexyl)-N-(3-pyridinyl)-2,3,5,6-tetrahydro-1*H*-cyclobuta[*f*]indol-1-carboxamid,
- 6-Cyano-6-(phenylsulfanyl)-N-(3-pyridinyl)-2,3,5,6-tetrahydro-1*H*-cyclobuta[*f*]indol-1-carboxamid,
- 6-Cyano-6-cyclohexyl-N-(3-pyridinyl)-2,3,5,6-tetrahydro-1*H*-cyclobuta[*f*]indol-1-carboxamid,
- 6-Cyclohexyl-N-(3-pyridinyl)-2,3,5,6-tetrahydro-1*H*-cyclobuta[*f*]indol-1-carboxamid,
- 6-Cyano-N-{6-[(2-methyl-3-pyridinyl)-oxy]-3-pyridinyl}-6-(phenylsulfanyl)-2,3,5,6-tetrahydro-1*H*-cyclobuta[*f*]indol-1-carboxamid,
deren Isomere sowie deren Additionssalze mit einer pharmazeutisch annehmbaren Säure oder Base.

9. Verfahren zur Herstellung der Verbindungen der Formel (I), **dadurch gekennzeichnet, daß** man als Ausgangsprodukt eine Verbindung der Formel (II) verwendet: 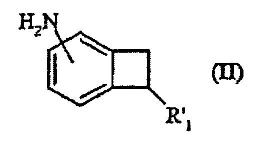 in der R'₁ eine Gruppe ausgewählt aus dem Wasserstoffatom, Cyano-, Hydroxymethylen-, Carboxy- und geradkettigen oder verzweigten (C₁-C₆)-Alkoxycarbonylgruppen bedeutet,
welche Verbindung der Formel (II) unter den Bedingungen der reduzierenden Aminierung mit einer Verbindung der Formel (**III**) umgesetzt wird:
(AO)₂CHCHO (**III**)
in der A eine geradkettige oder verzweigte (C₁-C₆)-Alkylgruppe darstellt, zur Bildung der Verbindungen der Formel (**IV**): 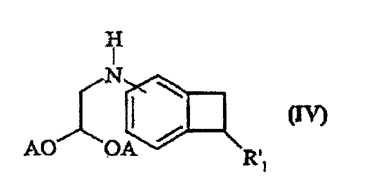 in der A und R'₁ die oben angegebenen Bedeutungen besitzen,
welche Verbindungen der Formel (IV) mit einer Verbindung der Formel (V) behandelt werden:
ClSO₂E (**V**)
in der E eine geradkettige oder verzweigte (C₁-C₄)-Alkylgruppe, Phenylgruppe oder p-Toluylgruppe bedeutet, zur Bildung der Verbindungen der Formel **(VI)**: 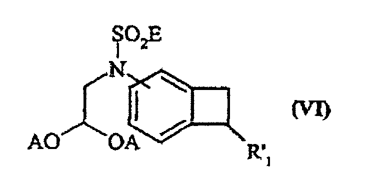 in der A,E und R'₁ die oben angegebenen Bedeutungen besitzen,
welche Verbindungen der Formel (VI) unter sauren Bedingungen cyclisiert werden zur Bildung der Verbindungen der Formel (VII): 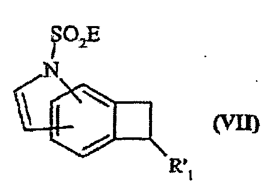 in der E und R'₁ die oben angegebenen Bedeutungen besitzen,
welche Verbindungen der Formel (VII) mit einem Alkalimetallhydroxid in einem alkoholischen Lösungsmittel oder mit Natrium in flüssigem Ammoniak behandelt werden zur Bildung der Verbindungen der Formel (VIII): 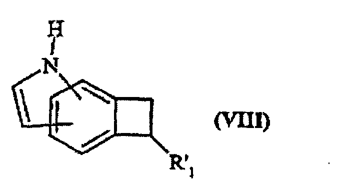 in der R'₁ die oben angegebenen Bedeutungen besitzt,
welche Verbindungen der Formel (VIII) anschließend unter klassischen Bedingungen der organischen Synthese reduziert werden zur Bildung der Verbindungen der Formel (**IX**): 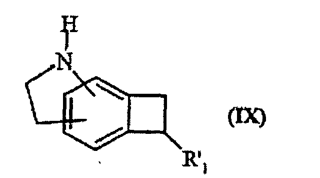 in der R'₁ die oben angegebenen Bedeutungen besitzt,
welche Verbindungen der Formel (IX) mit einem Isocyanat der Formel (**X**) behandelt werden:
R₃ - N = C = O (**X**)
in der R₃ die bezüglich der Formel (I) angegebenen Bedeutungen besitzt,
zur Bildung der Verbindungen der Formel **(I/a)**, einem Sonderfall der Verbindungen der Formel (I): 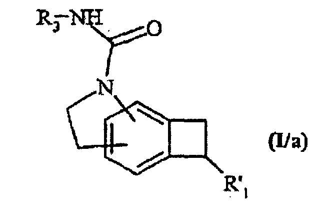 in der R'₁ und R₃ die oben angegebenen Bedeutungen besitzen,
oder man die Verbindungen der Formel (**IX**) in dem Fall, da R'₁ eine Cyanogruppe darstellt:
** entweder* mit einem Keton der Formel (**XI**) behandelt: 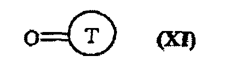 in der T die bezüglich der Formel (I) angegebenen Bedeutungen besitzt, zur Bildung der Verbindungen der Formel (**XII**): 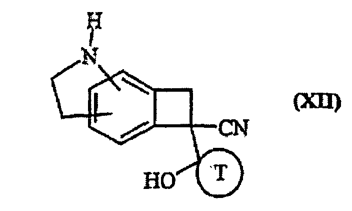 in der T die oben angegebenen Bedeutungen besitzt,
welche Verbindungen der Formel (XII) anschließend:
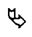 **entweder** mit einem Isocyanat der Formel (X), wie es oben definiert worden ist, behandelt werden zur Bildung der Verbindungen der Formel **(I/b)**, einem Sonderfall der Verbindungen der Formel (I): 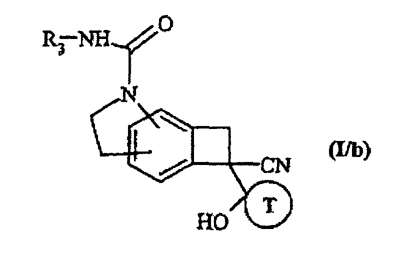 in der T und R₃ die oben angegebenen Bedeutungen besitzen,
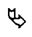 **oder** nach dem Schutz der Aminogruppe des Indolins gemäß den Methoden der klassischen organischen Synthese reduziert werden zur Bildung der Verbindungen der Formel (**XIII**): 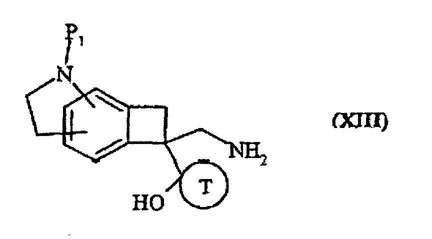 in der T die oben angegebenen Bedeutungen besitzt und P₁ eine klassische Schutzgruppe darstellt,
bei welchen Verbindungen der Formel (XIII) die primäre Aminfunktion unter Anwendung der üblichen Methoden der organischen Chemie anschließend substituiert wird und in eine sekundäre und dann tertiäre Aminfunktion umgewandelt wird zur Bildung der Verbindungen der Formel (**XIV**): 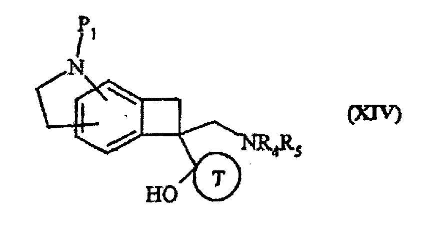 in der R₄ und R₅ die bezüglich der Formel (I) angegebenen Bedeutungen besitzen und T und P₁ die oben angegebenen Bedeutungen aufweisen,
welche Verbindungen der Formel (XIV) nach der Abspaltung der Schutzgruppe des Stickstoffatoms des Indolinkerns mit einer Verbindung der Formel (X), wie sie oben definiert worden ist, behandelt werden zur Bildung der Verbindungen der Formel **(I/c)**, einem Sonderfall der Verbindungen der Formel (I): 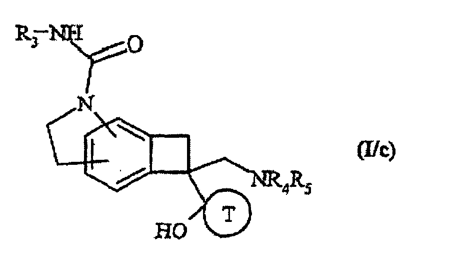 in der T, R₄, R₅ und R₃ die oben angegebenen Bedeutungen besitzen,
* *oder* mit einer starken Base oder einem Alkalimetallalkoholat in Gegenwart einer Verbindung der Formel **(XV)** behandelt:
W₁ - X **(XV)**
in der W₁ eine geradkettige oder verzweigte (C₁-C₆)-Alkylgruppe, geradkettige oder verzweigte Aryl-(C₁-C₆)-alkylgruppe, Cycloalkylgruppe oder geradkettige oder verzweigte Cycloalkyl-(C₁-C₆)-alkylgruppe und X eine austretende Gruppe, wie ein Halogenatom, eine Trifluormethylsulfonatgruppe, Mesylatgruppe oder Tosylatgruppe bedeuten,
zur Bildung der Verbindungen der Formel **(XVI)**: 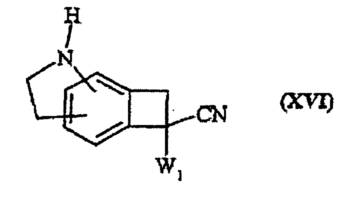 in der W₁ die oben angegebenen Bedeutungen besitzt,
welche Verbindungen der Formel (XVI):
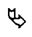 **entweder** mit einer Verbindung der Formel (X), wie sie oben definiert worden ist, behandelt werden zur Bildung der Verbindungen der Formel **(I/d)**, einem Sonderfall der Verbindungen der Formel (I): 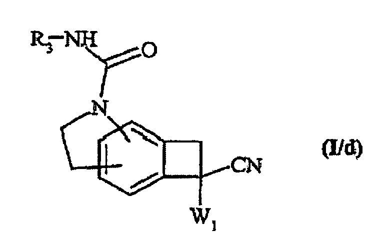 in der W₁ und R₃ die oben angegebenen Bedeutungen besitzen,
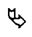 **oder** nach dem Abspalten der Schutzgruppe des Stickstoffatoms des Indolinkerns wie die Verbindungen der Formel (XII) in das primäre, sekundäre und dann tertiäre Amin umgewandelt werden, so daß nach dem Abspalten der Schutzgruppe und der Behandlung in Gegenwart einer Verbindung der Formel (X), wie sie oben definiert worden ist, die Verbindungen der Formel (**I/e**) erhalten werden, einem Sonderfall der Verbindungen der Formel (I): 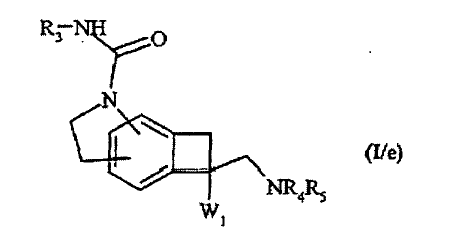 in der W₁, R₃, R₄ und R₅ die oben angegebenen Bedeutungen besitzen,
* *oder* mit Dibrom in einem organischen chlorhaltigen Lösungsmittel behandelt werden zur Bildung der Verbindungen der Formel (**XVII**): 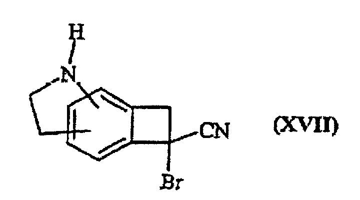 welche Verbindungen der Formel (XVII) mit einer Verbindung der Formel (**XVIII**) umgesetzt werden:
W - V₁ - H (**XVIII**)
in der W die bezüglich der Formel (I) angegebenen Bedeutungen besitzt und V₁ ein Sauerstoffatom oder ein Schwefelatom bedeutet,
zur Bildung der Verbindungen der Formel (**XIX**): 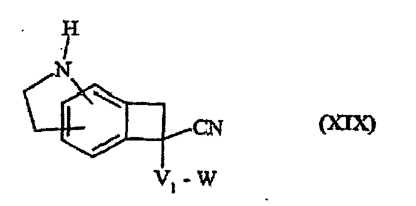 in der V₁ und W die oben angegebenen Bedeutungen besitzen,
welche Verbindungen der Formel (XIX):
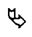 **entweder** mit einer Verbindung der Formel (X), wie sie oben definiert worden ist, behandelt werden zur Bildung der Verbindungen der Formel (I/f), einem Sonderfall der Verbindungen der Formel (I): 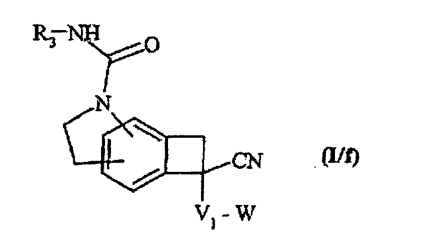 in der R₃, V₁ und W die oben angegebenen Bedeutungen besitzen,
welche Verbindungen der Formel (I/f) in dem besonderen Fall, da V₁ ein Schwefelatom darstellt, einer Oxidation unter klassischen Bedingungen der organischen Synthese unterworfen werden können zur Bildung der Verbindungen der Formel (**I/g**), einem Sonderfall der Verbindungen der Formel (I): 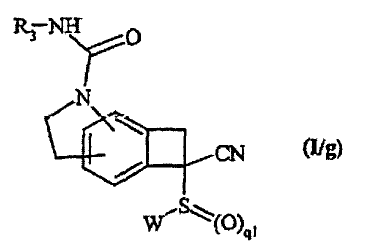 in der R₃ und W die bezüglich der Formel (I) angegebenen Bedeutungen besitzen und q₁ eine ganze Zahl mit einem Wert zwischen 1 und 2 einschließlich bedeutet,
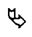 **oder** geschützt und dann unter Einhaltung der gleichen Folge von Reaktionen wie die Verbindungen der Formel (XII) in das primäre, sekundäre und tertiäre Amin umgewandelt werden, so daß nach dem Abspalten der Schutzgruppe und der Behandlung mit einer Verbindung der Formel (X), wie sie oben definiert worden ist, die Verbindungen der Formel (**I/h**) erhalten werden, einem Sonderfall der Verbindungen der Formel (I): 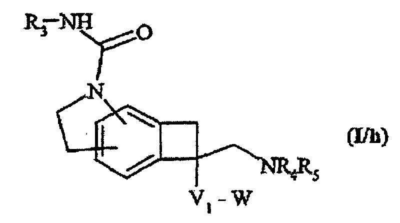 in der V₁, W, R₃, R₄ und R₅ die oben angegebenen Bedeutungen besitzen,
welche Verbindungen der Formel (I/h) in dem besonderen Fall, da V₁ ein Schwefelatom darstellt, einer Oxidation unter den klassischen Bedingungen der organischen Synthese unterworfen werden können zur Bildung der Verbindungen der Formel (**I/i**), einem Sonderfall der Verbindungen der Formel (I): 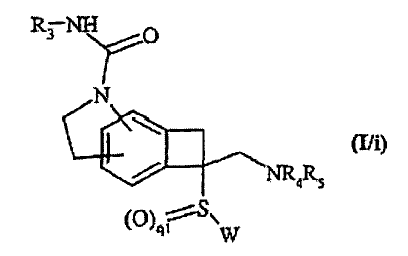 in der W, R₃, R₄, R₅ und q₁ die oben angegebenen Bedeutungen besitzen,
* *oder* mit einem Alkalimetallhydrid in Dimethylformamid in Gegenwart von Formaldehyd behandelt zur Bildung der Verbindungen der Formel **(XX)**: 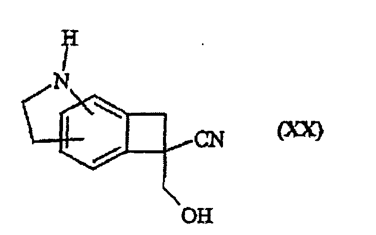 welche Verbindungen der Formel (XX):
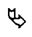 **entweder** mit einer Verbindung der Formel (X), wie sie oben definiert worden ist, behandelt werden zur Bildung der Verbindungen der Formel **(I/j)**, einem Sonderfall der Verbindungen der Formel (I): 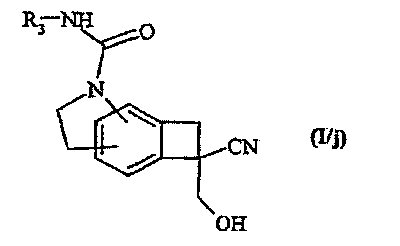 in der R₃ die bezüglich der Formel (I) angegebenen Bedeutungen besitzt,
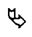 **oder** im Bereich des Stickstoffatoms des Indolinkerns geschützt und dann unter den Bedingungen der Mitsunobu-Reaktion mit einer Verbindung der Formel (**XXI**) behandelt werden:
W - OH (**XXI**)
in der W die bezüglich der Formel (I) angegebenen Bedeutungen besitzt,
und nach der Abspaltung der Stickstoffatoms des Indolkerns die Verbindungen der Formel (**XXII**) ergeben: 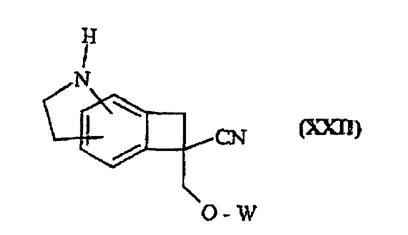 in der W die oben angegebenen Bedeutungen besitzt,
welche Verbindungen der Formel (XXII):
◆ *entweder* mit einer Verbindung der Formel (X), wie sie oben definiert worden ist, behandelt werden zur Bildung der Verbindungen der Formel (**I/k**), einem Sonderfall der Verbindungen der Formel (I): 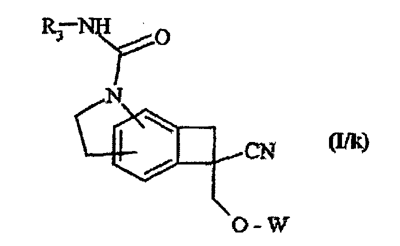 in der R₃ und W die oben angegebenen Bedeutungen besitzen,
◆ ***oder*** geschützt und dann wie die Verbindungen der Formel (XII) mit der gleichen Folge von Reaktionen in das primäre, sekundäre und tertiäre Amin umgewandelt werden, so daß nach der Abspaltung der Schutzgruppe und der Behandlung mit einer Verbindung der Formel (X), wie sie oben definiert worden ist, die Verbindungen der Formel (**I/l**) erhalten werden, einem Sonderfall der Verbindungen der Formel (I): 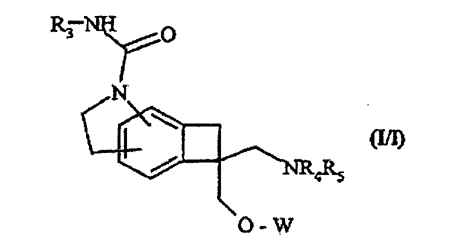 in der R₃, R₄, R₅ und W die oben angegebenen Bedeutungen besitzen,
welche Verbindungen der Formelm (I/a) bis (I/l), die die Gesamtheit der Verbindungen der Erfindung bilden, gegebenenfalls mit Hilfe einer klassischen Reinigungsmethode gereinigt werden, die gewünschtenfalls mit Hilfe einer klassischen Trennungstechnik in ihre verschiedenen Isomeren aufgetrennt werden können und die man gewünschtenfalls in ihre Additionssalze mit einer pharmazeutisch annehmbaren Säure oder Base überführt.

10. Pharmazeutische Zubereitungen enthaltend als Wirkstoff mindestens eine Verbindung der Formel (I) nach einem der Ansprüche 1 bis 9 allein oder in Kombination mit einem oder mehreren inerten, nichttoxischen, pharmazeutisch annehmbaren Trägermaterialien oder Bindemitteln.

11. Pharmazeutische Zubereitungen nach Anspruch 10, enthaltend mindestens einen Wirkstoff nach einem der Ansprüche 1 bis 9, nützlich als Arzneimittel bei der Behandlung der Angst, von Panikanfällen, von kompulsiven Besessenheitsstörungen, Phobien, impulsiven Störungen, des Drogenmißbrauchs, Erkenntnisstörungen, Psychosen, Depressionen und Gemütsstörungen.

## Claims

1. Compounds of formula **(I)** : 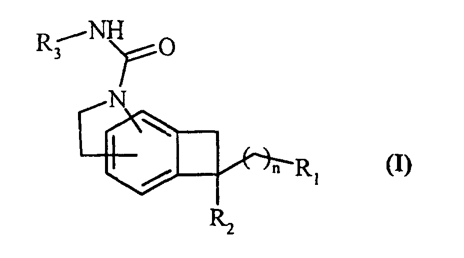 wherein :
***n*** represents an integer of from 0 to 6,
**R**_{**1**} represents a group selected from hydrogen, hydroxy, cyano, linear or branched (C₁-C₆)alkoxy, linear or branched (C₁-C₆)alkoxycarbonyl, carboxy, aminocarbonyl (the amino moiety optionally being substituted by one or two identical or different groups selected from linear or branched (C₁-C₆)alkyl, aryl and aryl-(C₁-C₆)alkyl in which the alkyl moiety may be linear or branched) and NR₄R₅ wherein R₄ and R₅, which may be identical or different, represent a group selected from linear or branched (C₁-C₆)alkyl, aryl, aryl-(C₁-C₆)alkyl in which the alkyl moiety may be linear or branched, heteroaryl, heteroaryl-(C₁-C₆)alkyl in which the alkyl moiety may be linear or branched, cycloalkyl, cycloalkyl-(C₁-C₆)alkyl in which the alkyl moiety may be linear or branched, linear or branched (C₂-C₆)alkenyl and linear or branched (C₂-C₆)alkynyl,
**R**_{**2**} represents a group selected from hydrogen, linear or branched (C₁-C₆)alkyl, hydroxymethyl, a group of formula 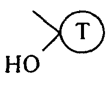 and -U-V-W
wherein :
* T represents a monocyclic or polycyclic (C₃-C₁₂)cycloalkyl group, it being possible for one of the carbon atoms of the cycloalkyl optionally to be replaced by a group selected from oxygen, selenium, a group of formula S(O)ₚ wherein p represents an integer of from 0 to 2 inclusive, and a group of formula SiR₆R₇ wherein R₆ and R₇, which may be identical or different, represent a linear or branched (C₁-C₆)alkyl group,
* U represents a bond or a methylene group,
* V represents a bond, an oxygen atom or a group S(O)_{q} wherein q is an integer of from 0 to 2 inclusive, and
* W represents a group selected from aryl, aryl-(C₁-C₆)alkyl in which the alkyl moiety may be linear or branched, cycloalkyl, and cycloalkyl-(C₁-C₆)alkyl in which the alkyl moiety may be linear or branched,
and
**R**_{**3**} represents a group selected from hydrogen, linear or branched (C₁-C₆)alkyl, aryl and heteroaryl,
their isomers, and also addition salts thereof with a pharmaceutically acceptable acid or base,
wherein:
• an aryl group is to be understood as a group selected from phenyl, biphenyl, naphthyl, dihydronaphthyl, tetrahydronaphthyl, indanyl, indenyl and benzocyclobutyl, each of which groups is optionally substituted by one or more identical or different groups selected from halogen atoms, linear or branched (C₁-C₆)alkyl, hydroxy, linear or branched (C₁-C₆)alkoxy, nitro, cyano, linear or branched (C₁-C₆)trihaloalkyl, amino, monoalkylamino, di-(C₁-C₆)alkylamino in which the alkyl moieties may be linear or branched, (C₁-C₆)trihaloalkoxy in which the alkoxy moiety may be linear or branched, amino-(C₁-C₆)alkylaminocarbonyl (the nitrogen atoms of each of the amino moieties optionally being substituted by identical or different linear or branched (C₁-C₆)alkyl groups), pyridyl, pyridyloxy and pyridyloxymethyl, the latter three groups optionally being substituted by a linear or branched (C₁-C₆)alkyl group,
• a heteroaryl group is to be understood as an aromatic monocyclic system, or a bicyclic system in which one of the rings is aromatic and the other ring is aromatic or partially hydrogenated, having from 5 to 12 ring members and containing one, two or three identical or different hetero atoms selected from oxygen, nitrogen and sulphur, each of the groups optionally being substituted by one or more identical or different groups selected from the substituents described for the aryl group defined above,
• and a cycloalkyl group is to be understood as a mono- or poly-cyclic system having from 3 to 12 ring members and optionally containing one or more unsaturated bonds,
wherein the unsaturated bonds do not confer an aromatic character on the said ring system.

2. Compounds of formula (I) according to claim 1, **characterised in that** R₂ represents a hydrogen atom, their isomers, and also addition salts thereof with a pharmaceutically acceptable acid or base.

3. Compounds of formula (I) according to claim 1, **characterised in that** R₂ represents a group of formula 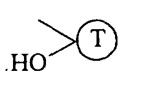 wherein T is as defined for formula (I), n is 1, and
R₁ represents a cyano group or an amino group substituted by one or two identical or different groups selected from linear or branched (C₁-C₆)alkyl and aryl-(C₁-C₆)alkyl in which the alkyl moiety may be linear or branched, their isomers, and also addition salts thereof with a pharmaceutically acceptable acid or base.

4. Compounds of formula (I) according to claim 1, **characterised in that** n is 0, R₁ represents a hydrogen atom or a cyano group and R₂ represents a hydrogen atom, their isomers, and also addition salts thereof with a pharmaceutically acceptable acid or base.

5. Compounds of formula (I) according to claim 1, **characterised in that** n is 0, R₁ represents a hydrogen atom or a cyano group and R₂ represents a group of formula -U-V-W wherein U represents a single bond, V represents a group of formula S(O)ₚ wherein p is as defined for formula (I) and W represents an aryl group, their isomers, and also addition salts thereof with a pharmaceutically acceptable acid or base.

6. Compounds of formula (I) according to claim 1, **characterised in that** R₃ represents a heteroaryl group, their isomers, and also addition salts thereof with a pharmaceutically acceptable acid or base.

7. Compounds of formula (I) according to claim 1, **characterised in that** R₃ represents a pyridyl group, their isomers, and also addition salts thereof with a pharmaceutically acceptable acid or base.

8. Compounds of formula (I) according to claim 1 that are :
- *N*-(3-pyridyl)-2,3,5,6-tetrahydro-1*H*-cyclobuta[*f*]indole-1-carboxamide,
- 5-cyano-*N*-(3-pyridyl)-2,3,5,6-tetrahydro-1*H*-cyclobuta[*f*]indole-1-carboxamide,
- 6-cyano-*N*-(3-pyridyl)-2,3,5,6-tetrahydro-1*H*-cyclobuta[*f*]indole-1-carboxamide,
- 6-(hydroxymethyl)-*N*-(3-pyridyl)-2,3,5,6-tetrahydro-1*H*-cyclobuta[*f*]indole-1-carboxamide,
- 5-(hydroxymethyl)-*N*-(3-pyridyl)-2,3,5,6-tetrahydro-1*H*-cyclobuta[*f*]indole-1-carboxamide,
- 7-cyano-*N*-(3-pyridyl)-1,2,6,7-tetrahydro-3*H*-cyclobuta[*e*]indole-3-carboxamide,
- 7-(hydroxymethyl)-*N*-(3-pyridyl)-2,3,6,7-tetrahydro-1*H*-cyclobuta[*g*]indole-1-carboxamide,
- 6-[(dimethylamino)methyl]-6-(1-hydroxycyclohexyl)-*N*-(3-pyridyl)-2,3,5,6-tetrahydro-1*H*-cyclobuta[*f*]indole-1-carboxamide,
- 6-cyano-6-(phenylsulphanyl)-*N*-(3-pyridyl)-2,3,5,6-tetrahydro-1*H*-cyclobuta[f]indole-1-carboxamide,
- 6-cyano-6-cyclohexyl-*N*-(3-pyridyl)-2,3,5,6-tetrahydro-1*H*-cyclobuta[f]indole-1-carboxamide,
- 6-cyclohexyl-*N*-(3-pyridyl)-2,3,5,6-tetrahydro-1*H*-cyclobuta[f]indole-1-carboxamide,
- 6-cyano-*N*-{6-[(2-methyl-3-pyridyl)oxy]-3-pyridyl}-6-(phenylsulphanyl)-2,3,5,6-tetrahydro-1*H*-cyclobuta[f]indole-1-carboxamide,
their isomers, and also addition salts thereof with a pharmaceutically acceptable acid or base.

9. Process for the preparation of the compounds of formula (I), **characterised in that** there is used as starting material a compound of formula **(II)** : 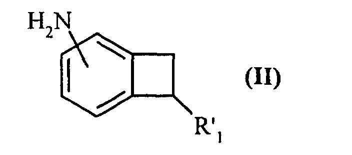 wherein R'₁ represents a group selected from hydrogen, cyano, hydroxymethylene, carboxy and linear or branched (C₁-C₆)alkoxycarbonyl,
which compound of formula (II) is reacted, under the conditions of reductive amination, with a compound of formula **(III)** :
(AO)₂CHCHO **(III)**
wherein A represents a linear or branched (C₁-C₆)alkyl group, to yield the compounds of formula **(IV)** : 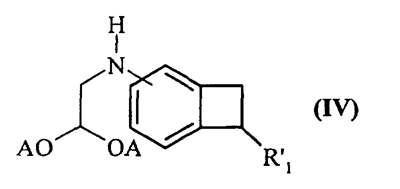 wherein A and R'₁ are as defined hereinbefore,
which compounds of formula (IV) are treated with a compound of formula **(V)** :
ClSO₂E **(V)**
wherein E represents a linear or branched (C₁-C₄)alkyl group, phenyl or p-toluyl, to yield the compounds of formula **(VI)** : 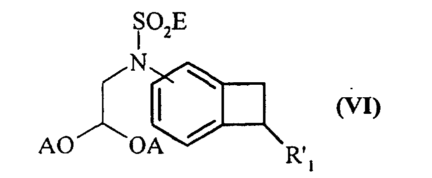 wherein A, E and R'₁ are as defined hereinbefore,
which compounds of formula (VI) are cyclised under acid conditions to yield the compounds of formula **(VII)** : 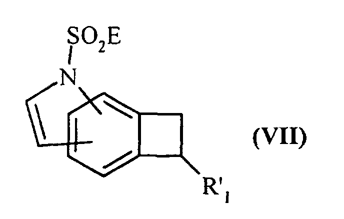 wherein E and R'₁ are as defined hereinbefore,
which compounds of formula (VII) are treated either with an alkali metal hydroxide in an alcoholic solvent or with sodium in liquid ammonia to yield the compounds of formula **(VIII)** : 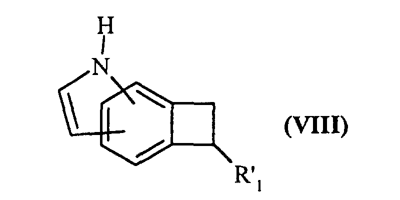 wherein R', is as defined hereinbefore,
which compounds of formula (VIII) are then reduced, in accordance with the conventional conditions of organic synthesis, to yield the compounds of formula **(IX)** : 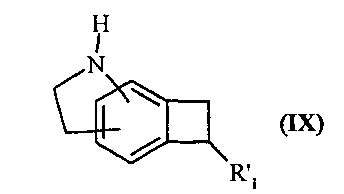 wherein R'₁ is as defined hereinbefore,
which compounds of formula (IX) are treated with an isocyanate of formula **(X)** :
R₃ - N = C = O **(X)**
wherein R₃ is as defined for formula (I),
to yield the compounds of formula **(I/a)**, a particular case of the compounds of formula (I): 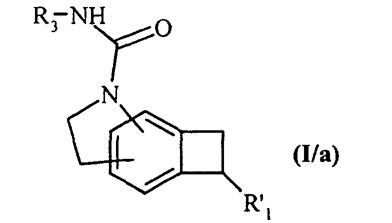 wherein R'₁ and R₃ are as defined hereinbefore,
or which compounds of formula **(IX)**, in the case where R'₁ represents a cyano group, are treated :
* *either* with a ketone of formula **(XI)** : 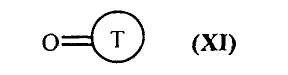 wherein T is as defined for formula (I), to yield the compounds of formula **(XII)** : 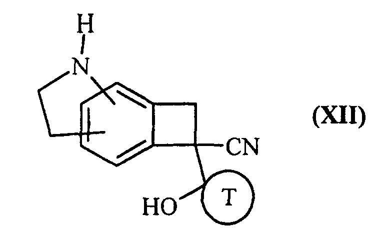 wherein T is as defined hereinbefore,
which compounds of formula (XII) are then :
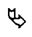 **either** treated with an isocyanate of formula (X) as described hereinbefore to yield the compounds of formula **(I/b)**, a particular case of the compounds of formula (I), 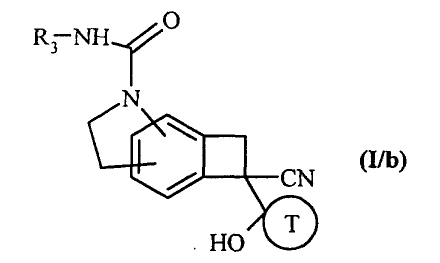 wherein T and R₃ are as defined hereinbefore,
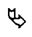 **or**, after protection of the amine of the indoline group, reduced according to the conventional methods of organic synthesis to yield the compounds of formula **(XIII)** : 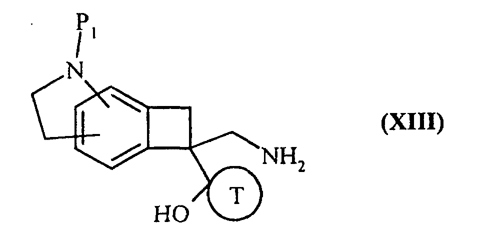 wherein T is as defined hereinbefore and P₁ is a conventional protecting group,
the primary amine function of which compounds of formula (XIII) is then substituted and converted to a secondary and then tertiary amine function, using conventional methods of organic chemistry, to yield the compounds of formula **(XIV)** : 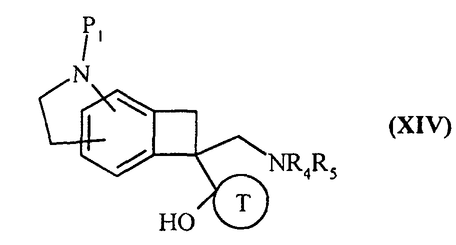 wherein R₄ and R₅ are as defined for formula (I) and T and P₁ are as defined hereinbefore,
which compounds of formula (XIV), after deprotection of the nitrogen atom of the indoline nucleus, are treated with a compound of formula (X) as described hereinbefore to yield the compounds of formula **(I/c)**, a particular case of the compounds of formula (I) : 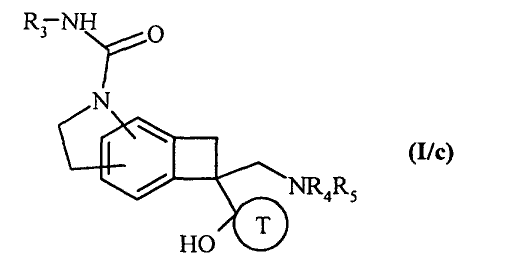 wherein T, R₄, R₅ and R₃ are as defined hereinbefore,
** or* with a strong base or an alkali metal alcoholate, in the presence of a compound of formula **(XV)** :
W₁ - X **(XV)**
wherein W₁ represents a linear or branched (C₁-C₆)alkyl group, an aryl-(C₁-C₆)alkyl group in which the alkyl moiety may be linear or branched, a cycloalkyl group, or a cycloalkyl-(C₁-C₆)alkyl group in which the alkyl moiety may be linear or branched, and X represents a leaving group, such as a halogen atom or a trifluoromethylsulphonate, mesylate or tosylate group,
to yield the compounds of formula (**XVI**) : 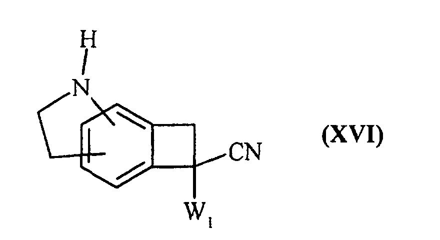 wherein W₁ is as defined hereinbefore,
which compounds of formula (XVI) are :
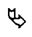 **either** treated with a compound of formula (X) as described hereinbefore to yield the compounds of formula **(I/d)**, a particular case of the compounds of formula (I) : 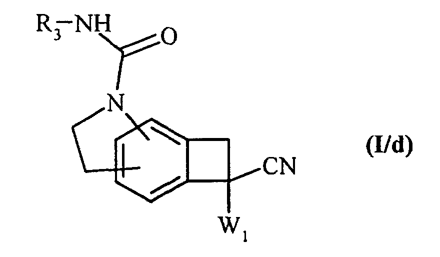 wherein W₁ and R₃ are as defined hereinbefore,
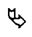 **or,** after protection of the nitrogen atom of the indoline nucleus, converted like the compounds of formula (XII) to a primary, secondary and then tertiary amine to yield, after deprotection and treatment in the presence of a compound of formula (X) as described hereinbefore, the compounds of formula **(I/e)**, a particular case of the compounds of formula (I) : 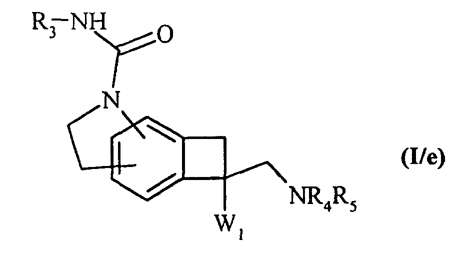 wherein W₁, R₃, R₄ and R₅ are as defined hereinbefore,
** or* with bromine in a chlorine-containing organic solvent to yield the compounds of formula **(XVII)** : 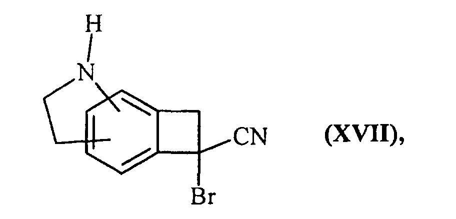 which compounds of formula (XVII) are reacted with a compound of formula **(XVIII)** :
W - V₁ - H **(XVIII)**
wherein W is as defined for formula (I) and V₁ represents an oxygen atom or a sulphur atom,
to yield the compounds of formula **(XIX)** : 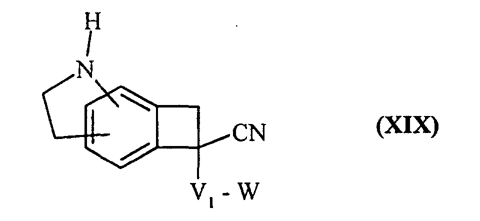 wherein V₁ and W are as defined hereinbefore,
which compounds of formula (XIX) are :
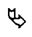 **either** treated with a compound of formula (X) as described hereinbefore to yield the compounds of formula **(I/f)**, a particular case of the compounds of formula (I) : 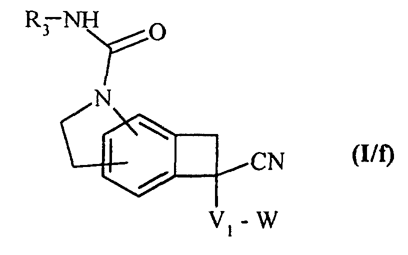 wherein R₃, V, and W are as defined hereinbefore,
which compounds of formula (I/f), in the case where V₁ represents a sulphur atom, may be subjected to oxidation under conventional conditions of organic synthesis to yield the compounds of formula **(I/g)**, a particular case of the compounds of formula (I) : 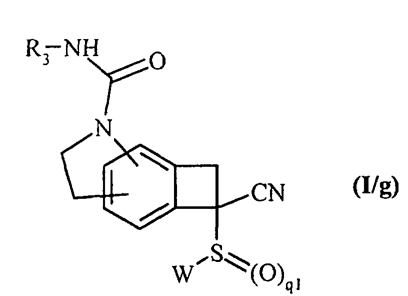 wherein R₃ and W are as defined for formula (I) and q₁ is an integer of from 1 to 2 inclusive,
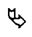 **or** protected and then converted, by the same sequence of reactions as the compounds of formula (XII), to a primary, secondary and tertiary amine to yield, after deprotection and treatment with a compound of formula (X), as described hereinbefore, the compounds of formula **(I/h)**, a particular case of the compounds of formula (I): 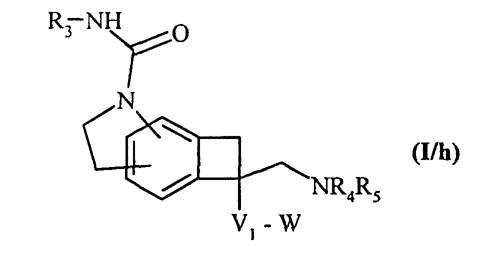 wherein V₁, W, R₃, R₄ and R₅ are as defined hereinbefore,
which compounds of formula (I/h), in the case where V, represents a sulphur atom, may be subjected to oxidation under conventional conditions of organic synthesis to yield the compounds of formula **(I/i)**, a particular case of the compounds of formula (I) : 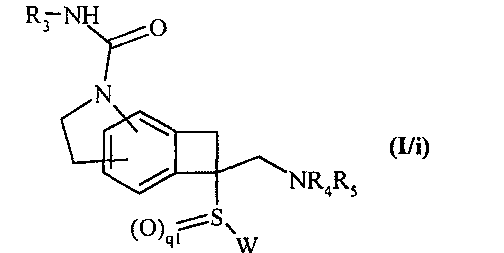 wherein W, R₃, R₄, R₅ and q₁ are as defined hereinbefore,
* or with an alkali metal hydride in dimethylformamide, in the presence of formaldehyde, to yield the compounds of formula **(XX)** : 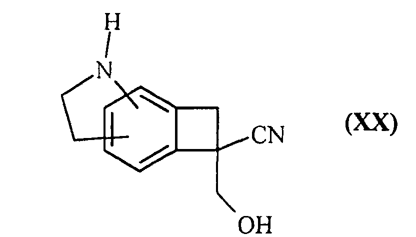 which compounds of formula (XX) are :
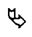 **either** treated with a compound of formula (X) as described hereinbefore to yield the compounds of formula **(I/j)**, a particular case of the compounds of formula (I) : 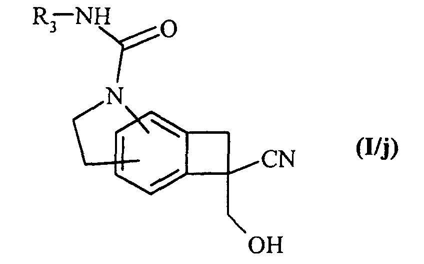 wherein R₃ is as defined for formula (I),
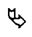 **or** protected at the nitrogen atom of the indoline nucleus, then treated according to Mitsunobu reaction conditions with a compound of formula **(XXI)** :
W - OH **(XXI)**
wherein W is as defined for formula (I),
to yield, after deprotection of the nitrogen atom of the indoline nucleus, the compounds of formula **(XXII)** : 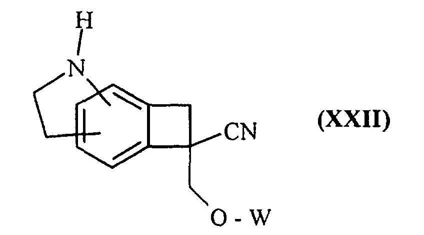 wherein W is as defined hereinbefore,
which compounds of formula (XXII) are :
**◆ *either*** treated with a compound of formula (X) as described hereinbefore to yield the compounds of formula **(I/k)**, a particular case of the compounds of formula (I) : 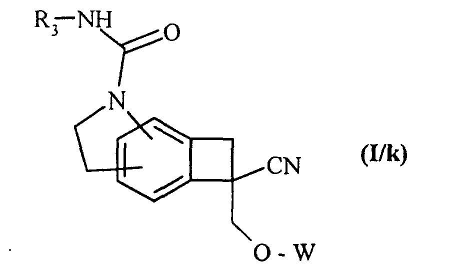 wherein R₃ and W are as defined hereinbefore,
**◆ *or*** protected and then converted, by the same reaction sequence as the compounds of formula (XII), to a primary, secondary and tertiary amine to yield, after deprotection and treatment with a compound of formula (X), as described hereinbefore, the compounds of formula **(I/l)**, a particular case of the compounds of formula (I) : 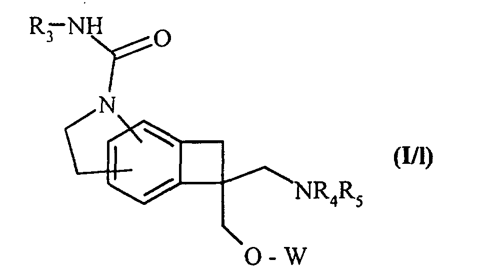 wherein R₃, R₄, R₅ and W are as defined hereinbefore,
the compounds (I/a) to (I/l) constituting the totality of the compounds of the invention, which compounds are purified, if necessary, according to a conventional purification technique, may be separated, if desired, into their different isomers according to a conventional separation technique, and are converted, if desired, into addition salts with a pharmaceutically acceptable acid or base.

10. Pharmaceutical compositions comprising as active ingredient at least one compound of formula (I) according to any one of claims I to 9, alone or in combination with one or more pharmaceutically acceptable, inert, non-toxic excipients or carriers.

11. Pharmaceutical compositions according to claim 10 containing at least one active ingredient according to any one of claims 1 to 9 for use as medicaments in the treatment of anxiety, panic attacks, obsessive-compulsive disorders, phobias, impulsive disorders, drug abuse, cognitive disorders, psychoses, depression and mood disorders.
